# EUROPEAN PATENT SPECIFICATION

(11) **EP 3 485 034 B1**
(45) Date of publication and mention of the grant of the patent: **18.08.2021**
(21) Application number: 17743157.4
(22) Date of filing: 12.07.2017
(51) Int. Cl.: C12Q 1/68

(54) **SYSTEM AND METHOD FOR TRANSPOSASE-MEDIATED AMPLICON SEQUENCING**
SYSTEM UND VERFAHREN ZUR TRANSPOSASE-VERMITTELTEN AMPLICON-SEQUENZIERUNG
SYSTÈME ET PROCÉDÉ DE SÉQUENÇAGE D'AMPLICON MÉDIÉ PAR UNE TRANSPOSASE

(30) Priority: 12.07.2016 US 201662361347 P; 30.09.2016 US 201662402523 P
(43) Date of publication of application: 22.05.2019
(73) Proprietor: Kapa Biosystems, Inc., Wilmington, MA 01887 (US)
(72) Inventor: MILLER, Bronwen, 8005 Sea Point Cape Town (ZA); VAN DER WALT, Eric, 7700 Newlands Cape Town (ZA); PENKLER, Jo-Anne Elizabeth, 7975 Fish Hoek (ZA); RANIK, Martin, 7200 Sandbaai, Hermanus (ZA)
(74) Representative: Hildebrandt, Martin K. E.
(86) International application number: PCT/US2017/041771
(87) International publication number: WO 2018/013724

(56) References cited:
- WO-A1-2016/014409
- WO-A2-2016/033251
- WO-A2-2016/061517
- Anonymous: "Nextera Enrichment Sample Preparation Guide", Illumina , February 2013 (2013-02), pages FP-V, 1-64, XP002774284, Retrieved from the Internet: URL:https://support.illumina.com/content/d am/illumina-support/documents/documentatio n/chemistry_documentation/samplepreps_next era/nexteraenrichment/nextera_enrichment_s ampleprep_guide_15032301_b.pdf [retrieved on 2017-09-29]

### SEQUENCE LISTING

<110> KAPA BIOSYSTEMS, INC.
<120> SYSTEM AND METHOD FOR TRANSPOSASE-MEDIATED AMPLICON SEQUENCING
<130> RMSI-011/001WO
<140>
   <141>
<150> 62/402,523
   <151> 2016-09-30
<150> 62/361,347
   <151> 2016-07-12
<160> 152
<170> PatentIn version 3.5
<210> 1
   <211> 33
   <212> DNA
   <213> Artificial Sequence
<220>
   <223> Description of Artificial Sequence: Synthetic oligonucleotide
<400> 1
   tcgtcggcag cgtcagatgt gtataagaga cag 33
<210> 2
   <211> 51
   <212> DNA
   <213> Artificial Sequence
<220>
   <223> Description of Artificial Sequence: Synthetic primer
<400> 2
   aatgatacgg cgaccaccga gatctacacg cgtaagatcg tcggcagcgt c 51
<210> 3
   <211> 25
   <212> DNA
   <213> Artificial Sequence
<220>
   <223> Description of Artificial Sequence: Synthetic primer
<400> 3
   cttctccact taataagcag ttgat 25
<210> 4
   <211> 30
   <212> DNA
   <213> Artificial Sequence
<220>
   <223> Description of Artificial Sequence: Synthetic primer
<400> 4
   taaggtatca ataaatactc accaatcttc 30
<210> 5
   <211> 17
   <212> DNA
   <213> Artificial Sequence
<220>
   <223> Description of Artificial Sequence: Synthetic primer
<400> 5
   ccaggctgcc tcaccat 17
<210> 6
   <211> 60
   <212> DNA
   <213> Artificial Sequence
<220>
   <223> Description of Artificial Sequence: Synthetic primer
<400> 6
   gtctcgtggg ctcggagatg tgtataagag acagtaccag tgtgttattc aggtaggtca 60
<210> 7
   <211> 64
   <212> DNA
   <213> Artificial Sequence
<220>
   <223> Description of Artificial Sequence: Synthetic primer
<400> 7
   gtctcgtggg ctcggagatg tgtataagag acagaatttt caactgcttt acataagaag 60
cgtt 64
<210> 8
   <211> 53
   <212> DNA
   <213> Artificial Sequence
<220>
   <223> Description of Artificial Sequence: Synthetic primer
<400> 8
   gtctcgtggg ctcggagatg tgtataagag acaggtcctg actgtggcgt cat 53
<210> 9
   <211> 39
   <212> DNA
   <213> Artificial Sequence
<220>
   <223> Description of Artificial Sequence: Synthetic primer
<400> 9
   caagcagaag acggcatacg agatgtctcg tgggctcgg 39
<210> 10
   <211> 33
   <212> DNA
   <213> Artificial Sequence
<220>
   <223> Description of Artificial Sequence: Synthetic oligonucleotide
<400> 10
   tcgtcggcag cgtcagatgt gtataagaga cag 33
<210> 11
   <211> 19
   <212> DNA
   <213> Artificial Sequence
<220>
   <223> Description of Artificial Sequence: Synthetic oligonucleotide
<400> 11
   ctgtctctta tacacatct 19
<210> 12
   <211> 51
   <212> DNA
   <213> Artificial Sequence
<220>
   <223> Description of Artificial Sequence: Synthetic primer
<400> 12
   aatgatacgg cgaccaccga gatctacact agatcgctcg tcggcagcgt c 51
<210> 13
   <211> 59
   <212> DNA
   <213> Artificial Sequence
<220>
   <223> Description of Artificial Sequence: Synthetic oligonucleotide
<220>
   <221> modified-base
   <222> (19)..(26)
   <223> a, c, t, g, unknown or other
<400> 13
   gaccaccgag atctacacnn nnnnnntcgt cggcagcgtc agatgtgtat aagagacag 59
<210> 14
   <211> 19
   <212> DNA
   <213> Artificial Sequence
<220>
   <223> Description of Artificial Sequence: Synthetic oligonucleotide
<400> 14
   ctgtctctta tacacatct 19
<210> 15
   <211> 29
   <212> DNA
   <213> Artificial Sequence
<220>
   <223> Description of Artificial Sequence: Synthetic primer
<400> 15
   aatgatacgg cgaccaccga gatctacac 29
<210> 16
   <211> 20
   <212> DNA
   <213> Artificial Sequence
<220>
   <223> Description of Artificial Sequence: Synthetic primer
<400> 16
   aatgatacgg cgaccaccga 20
<210> 17
   <211> 23
   <212> DNA
   <213> Artificial Sequence
<220>
   <223> Description of Artificial Sequence: Synthetic primer
<400> 17
   cagaaatcct aaatggtgga gtc 23
<210> 18
   <211> 19
   <212> DNA
   <213> Artificial Sequence
<220>
   <223> Description of Artificial Sequence: Synthetic primer
<400> 18
   ggcttgggca aaggaaata 19
<210> 19
   <211> 20
   <212> DNA
   <213> Artificial Sequence
<220>
   <223> Description of Artificial Sequence: Synthetic primer
<400> 19
   caaggaagca ggacaccaat 20
<210> 20
   <211> 22
   <212> DNA
   <213> Artificial Sequence
<220>
   <223> Description of Artificial Sequence: Synthetic primer
<400> 20
   tcaaacatca tcttgtgaaa ca 22
<210> 21
   <211> 22
   <212> DNA
   <213> Artificial Sequence
<220>
   <223> Description of Artificial Sequence: Synthetic primer
<400> 21
   cagttggctt actggaagtt ga 22
<210> 22
   <211> 24
   <212> DNA
   <213> Artificial Sequence
<220>
   <223> Description of Artificial Sequence: Synthetic primer
<400> 22
   aatcggttta ggaatacaat tctg 24
<210> 23
   <211> 26
   <212> DNA
   <213> Artificial Sequence
<220>
   <223> Description of Artificial Sequence: Synthetic primer
<400> 23
   ccagaattat aggaacttgc taacag 26
<210> 24
   <211> 21
   <212> DNA
   <213> Artificial Sequence
<220>
   <223> Description of Artificial Sequence: Synthetic primer
<400> 24
   ggtggaggta attttgaagc a 21
<210> 25
   <211> 21
   <212> DNA
   <213> Artificial Sequence
<220>
   <223> Description of Artificial Sequence: Synthetic primer
<400> 25
   caaggggaaa gtgtaaatca a 21
<210> 26
   <211> 20
   <212> DNA
   <213> Artificial Sequence
<220>
   <223> Description of Artificial Sequence: Synthetic primer
<400> 26
   ccccatggaa cttaccaagc 20
<210> 27
   <211> 22
   <212> DNA
   <213> Artificial Sequence
<220>
   <223> Description of Artificial Sequence: Synthetic primer
<400> 27
   aaaaccatct ttcgtttcct tc 22
<210> 28
   <211> 18
   <212> DNA
   <213> Artificial Sequence
<220>
   <223> Description of Artificial Sequence: Synthetic primer
<400> 28
   cattttggcc aggatgat 18
<210> 29
   <211> 20
   <212> DNA
   <213> Artificial Sequence
<220>
   <223> Description of Artificial Sequence: Synthetic primer
<400> 29
   ttcaaagcca tttttccaga 20
<210> 30
   <211> 17
   <212> DNA
   <213> Artificial Sequence
<220>
   <223> Description of Artificial Sequence: Synthetic primer
<400> 30
   gtgggaaggc ggtgttg 17
<210> 31
   <211> 18
   <212> DNA
   <213> Artificial Sequence
<220>
   <223> Description of Artificial Sequence: Synthetic primer
<400> 31
   cacagcgtct ccgagtcc 18
<210> 32
   <211> 24
   <212> DNA
   <213> Artificial Sequence
<220>
   <223> Description of Artificial Sequence: Synthetic primer
<400> 32
   gcacagttca gaggatattt aagc 24
<210> 33
   <211> 18
   <212> DNA
   <213> Artificial Sequence
<220>
   <223> Description of Artificial Sequence: Synthetic primer
<400> 33
   gtgcagccct cagggagt 18
<210> 34
   <211> 25
   <212> DNA
   <213> Artificial Sequence
<220>
   <223> Description of Artificial Sequence: Synthetic primer
<400> 34
   cacccccagg attcttacag aaaac 25
<210> 35
   <211> 23
   <212> DNA
   <213> Artificial Sequence
<220>
   <223> Description of Artificial Sequence: Synthetic primer
<400> 35
   ctgccagaca tgagaaaagg tgg 23
<210> 36
   <211> 25
   <212> DNA
   <213> Artificial Sequence
<220>
   <223> Description of Artificial Sequence: Synthetic primer
<400> 36
   aatatacagc ttgcaaggac tctgg 25
<210> 37
   <211> 25
   <212> DNA
   <213> Artificial Sequence
<220>
   <223> Description of Artificial Sequence: Synthetic primer
<400> 37
   ccaatattgt ctttgtgttc ccgga 25
<210> 38
   <211> 29
   <212> DNA
   <213> Artificial Sequence
<220>
   <223> Description of Artificial Sequence: Synthetic primer
<400> 38
   tctgctttat ttattccaat aggtatggt 29
<210> 39
   <211> 26
   <212> DNA
   <213> Artificial Sequence
<220>
   <223> Description of Artificial Sequence: Synthetic primer
<400> 39
   agttgaaact aaaaatcctt tgcagg 26
<210> 40
   <211> 25
   <212> DNA
   <213> Artificial Sequence
<220>
   <223> Description of Artificial Sequence: Synthetic primer
<400> 40
   taaacaatac agctagtggg aaggc 25
<210> 41
   <211> 27
   <212> DNA
   <213> Artificial Sequence
<220>
   <223> Description of Artificial Sequence: Synthetic primer
<400> 41
   agtgtattaa ccttatgtgt gacatgt 27
<210> 42
   <211> 25
   <212> DNA
   <213> Artificial Sequence
<220>
   <223> Description of Artificial Sequence: Synthetic primer
<400> 42
   tgagtgaagg actgagaaaa tccct 25
<210> 43
   <211> 31
   <212> DNA
   <213> Artificial Sequence
<220>
   <223> Description of Artificial Sequence: Synthetic primer
<400> 43
   agaaattaga tctcttacct aaactcttca t 31
<210> 44
   <211> 25
   <212> DNA
   <213> Artificial Sequence
<220>
   <223> Description of Artificial Sequence: Synthetic primer
<400> 44
   gtggaatcca gagtgagctt tcatt 25
<210> 45
   <211> 25
   <212> DNA
   <213> Artificial Sequence
<220>
   <223> Description of Artificial Sequence: Synthetic primer
<400> 45
   gttaatcaac tgatgcaaac tcttg 25
<210> 46
   <211> 25
   <212> DNA
   <213> Artificial Sequence
<220>
   <223> Description of Artificial Sequence: Synthetic primer
<400> 46
   aaataatgct cctagtacct gtaga 25
<210> 47
   <211> 28
   <212> DNA
   <213> Artificial Sequence
<220>
   <223> Description of Artificial Sequence: Synthetic primer
<400> 47
   tcctttaata cagaatatgg gtaaagat 28
<210> 48
   <211> 22
   <212> DNA
   <213> Artificial Sequence
<220>
   <223> Description of Artificial Sequence: Synthetic primer
<400> 48
   tgtaaacctt gcagacaaac tc 22
<210> 49
   <211> 19
   <212> DNA
   <213> Artificial Sequence
<220>
   <223> Description of Artificial Sequence: Synthetic primer
<400> 49
   ccatgaggca gagcatacg 19
<210> 50
   <211> 19
   <212> DNA
   <213> Artificial Sequence
<220>
   <223> Description of Artificial Sequence: Synthetic primer
<400> 50
   acatcctggt agctgaggg 19
<210> 51
   <211> 30
   <212> DNA
   <213> Artificial Sequence
<220>
   <223> Description of Artificial Sequence: Synthetic primer
<400> 51
   gtcttttggt ttttcttgat agtattaatg 30
<210> 52
   <211> 25
   <212> DNA
   <213> Artificial Sequence
<220>
   <223> Description of Artificial Sequence: Synthetic primer
<400> 52
   ttcttcctaa gtgcaaaaga taact 25
<210> 53
   <211> 30
   <212> DNA
   <213> Artificial Sequence
<220>
   <223> Description of Artificial Sequence: Synthetic primer
<400> 53
   cagtgtttct tttaaatacc tgttaagttt 30
<210> 54
   <211> 22
   <212> DNA
   <213> Artificial Sequence
<220>
   <223> Description of Artificial Sequence: Synthetic primer
<400> 54
   ccacagttgc acaatatcct tt 22
<210> 55
   <211> 21
   <212> DNA
   <213> Artificial Sequence
<220>
   <223> Description of Artificial Sequence: Synthetic primer
<400> 55
   tgcagcaatt cactgtaaag c 21
<210> 56
   <211> 30
   <212> DNA
   <213> Artificial Sequence
<220>
   <223> Description of Artificial Sequence: Synthetic primer
<400> 56
   ctaatgtata tatgttctta aatggctacg 30
<210> 57
   <211> 19
   <212> DNA
   <213> Artificial Sequence
<220>
   <223> Description of Artificial Sequence: Synthetic primer
<400> 57
   ccaggaccag aggaaacct 19
<210> 58
   <211> 29
   <212> DNA
   <213> Artificial Sequence
<220>
   <223> Description of Artificial Sequence: Synthetic primer
<400> 58
   aaatagttta agatgagtca tatttgtgg 29
<210> 59
   <211> 18
   <212> DNA
   <213> Artificial Sequence
<220>
   <223> Description of Artificial Sequence: Synthetic primer
<400> 59
   ctgtggggtg gagagctg 18
<210> 60
   <211> 19
   <212> DNA
   <213> Artificial Sequence
<220>
   <223> Description of Artificial Sequence: Synthetic primer
<400> 60
   ggtcacactt gttccccac 19
<210> 61
   <211> 18
   <212> DNA
   <213> Artificial Sequence
<220>
   <223> Description of Artificial Sequence: Synthetic primer
<400> 61
   ccgtttgatc tgctccct 18
<210> 62
   <211> 18
   <212> DNA
   <213> Artificial Sequence
<220>
   <223> Description of Artificial Sequence: Synthetic primer
<400> 62
   ctggatggtc agcgcact 18
<210> 63
   <211> 26
   <212> DNA
   <213> Artificial Sequence
<220>
   <223> Description of Artificial Sequence: Synthetic primer
<400> 63
   gttatgattt tgcagaaaac agatct 26
<210> 64
   <211> 29
   <212> DNA
   <213> Artificial Sequence
<220>
   <223> Description of Artificial Sequence: Synthetic primer
<400> 64
   actataatta ctccttaatg tcagcttat 29
<210> 65
   <211> 26
   <212> DNA
   <213> Artificial Sequence
<220>
   <223> Description of Artificial Sequence: Synthetic primer
<400> 65
   tgaaaatggt cagagaaacc tttatc 26
<210> 66
   <211> 23
   <212> DNA
   <213> Artificial Sequence
<220>
   <223> Description of Artificial Sequence: Synthetic primer
<400> 66
   tgcagttgtt taccatgata acg 23
<210> 67
   <211> 30
   <212> DNA
   <213> Artificial Sequence
<220>
   <223> Description of Artificial Sequence: Synthetic primer
<400> 67
   cgaaataaca caaattttta aggttactga 30
<210> 68
   <211> 23
   <212> DNA
   <213> Artificial Sequence
<220>
   <223> Description of Artificial Sequence: Synthetic primer
<400> 68
   tgattacaca gtatcctcga cat 23
<210> 69
   <211> 27
   <212> DNA
   <213> Artificial Sequence
<220>
   <223> Description of Artificial Sequence: Synthetic primer
<400> 69
   ctgaaaagag tgaaggatat aggatac 27
<210> 70
   <211> 22
   <212> DNA
   <213> Artificial Sequence
<220>
   <223> Description of Artificial Sequence: Synthetic primer
<400> 70
   actgttcttc ctcagacatt ca 22
<210> 71
   <211> 21
   <212> DNA
   <213> Artificial Sequence
<220>
   <223> Description of Artificial Sequence: Synthetic primer
<400> 71
   ccacagagaa gttgttgagg g 21
<210> 72
   <211> 20
   <212> DNA
   <213> Artificial Sequence
<220>
   <223> Description of Artificial Sequence: Synthetic primer
<400> 72
   gccttgtact gcagagacaa 20
<210> 73
   <211> 18
   <212> DNA
   <213> Artificial Sequence
<220>
   <223> Description of Artificial Sequence: Synthetic primer
<400> 73
   tggtgatggg cttggtcc 18
<210> 74
   <211> 19
   <212> DNA
   <213> Artificial Sequence
<220>
   <223> Description of Artificial Sequence: Synthetic primer
<400> 74
   tacagcggag aagggagcg 19
<210> 75
   <211> 18
   <212> DNA
   <213> Artificial Sequence
<220>
   <223> Description of Artificial Sequence: Synthetic primer
<400> 75
   caggtaggat ccagccca 18
<210> 76
   <211> 18
   <212> DNA
   <213> Artificial Sequence
<220>
   <223> Description of Artificial Sequence: Synthetic primer
<400> 76
   atcccccaaa gccaacaa 18
<210> 77
   <211> 18
   <212> DNA
   <213> Artificial Sequence
<220>
   <223> Description of Artificial Sequence: Synthetic primer
<400> 77
   gtttgggggt gtgtggtc 18
<210> 78
   <211> 18
   <212> DNA
   <213> Artificial Sequence
<220>
   <223> Description of Artificial Sequence: Synthetic primer
<400> 78
   ggtgcttccc attccagg 18
<210> 79
   <211> 20
   <212> DNA
   <213> Artificial Sequence
<220>
   <223> Description of Artificial Sequence: Synthetic primer
<400> 79
   acagtcaaga agaaaacggc 20
<210> 80
   <211> 19
   <212> DNA
   <213> Artificial Sequence
<220>
   <223> Description of Artificial Sequence: Synthetic primer
<400> 80
   agaggagctg gtgttgttg 19
<210> 81
   <211> 18
   <212> DNA
   <213> Artificial Sequence
<220>
   <223> Description of Artificial Sequence: Synthetic primer
<400> 81
   accgcttctt gtcctgct 18
<210> 82
   <211> 20
   <212> DNA
   <213> Artificial Sequence
<220>
   <223> Description of Artificial Sequence: Synthetic primer
<400> 82
   gagcagtaag gagattcccc 20
<210> 83
   <211> 19
   <212> DNA
   <213> Artificial Sequence
<220>
   <223> Description of Artificial Sequence: Synthetic primer
<400> 83
   aaatcggtaa gaggtgggc 19
<210> 84
   <211> 18
   <212> DNA
   <213> Artificial Sequence
<220>
   <223> Description of Artificial Sequence: Synthetic primer
<400> 84
   ccccctactg ctcacctg 18
<210> 85
   <211> 61
   <212> DNA
   <213> Artificial Sequence
<220>
   <223> Description of Artificial Sequence: Synthetic primer
<400> 85
   gtctcgtggg ctcggagatg tgtataagag acagagaaca aattcctttg ttatgcagac 60
   a 61
<210> 86
   <211> 64
   <212> DNA
   <213> Artificial Sequence
<220>
   <223> Description of Artificial Sequence: Synthetic primer
<400> 86
   gtctcgtggg ctcggagatg tgtataagag acagttgcca atatttaacc aattttgacc 60
   taca 64
<210> 87
   <211> 63
   <212> DNA
   <213> Artificial Sequence
<220>
   <223> Description of Artificial Sequence: Synthetic primer
<400> 87
   gtctcgtggg ctcggagatg tgtataagag acagcaagaa gatgctctga gtctaatgaa 60
   gtt 63
<210> 88
   <211> 64
   <212> DNA
   <213> Artificial Sequence
<220>
   <223> Description of Artificial Sequence: Synthetic primer
<400> 88
   gtctcgtggg ctcggagatg tgtataagag acagggtcta taatccagat gattctttaa 60
   cagg 64
<210> 89
   <211> 56
   <212> DNA
   <213> Artificial Sequence
<220>
   <223> Description of Artificial Sequence: Synthetic primer
<400> 89
   gtctcgtggg ctcggagatg tgtataagag acagctttgt ccacctggaa cttggt 56
<210> 90
   <211> 64
   <212> DNA
   <213> Artificial Sequence
<220>
   <223> Description of Artificial Sequence: Synthetic primer
<400> 90
   gtctcgtggg ctcggagatg tgtataagag acagctcact ctaacaagca gataactttc 60
   actt 64
<210> 91
   <211> 61
   <212> DNA
   <213> Artificial Sequence
<220>
   <223> Description of Artificial Sequence: Synthetic primer
<400> 91
   gtctcgtggg ctcggagatg tgtataagag acagcagaga atgagggagg agtacattac 60
t 61
<210> 92
   <211> 61
   <212> DNA
   <213> Artificial Sequence
<220>
   <223> Description of Artificial Sequence: Synthetic primer
<400> 92
   gtctcgtggg ctcggagatg tgtataagag acagtttttg ctttacgtga tgactttgtt 60
   g 61
<210> 93
   <211> 63
   <212> DNA
   <213> Artificial Sequence
<220>
   <223> Description of Artificial Sequence: Synthetic primer
<400> 93
   gtctcgtggg ctcggagatg tgtataagag acaggcaaac cacaaaagta tactccatgg 60
   tta 63
<210> 94
   <211> 58
   <212> DNA
   <213> Artificial Sequence
<220>
   <223> Description of Artificial Sequence: Synthetic primer
<400> 94
   gtctcgtggg ctcggagatg tgtataagag acagtccatc tcttggaaac tcccatct 58
<210> 95
   <211> 62
   <212> DNA
   <213> Artificial Sequence
<220>
   <223> Description of Artificial Sequence: Synthetic primer
<400> 95
   gtctcgtggg ctcggagatg tgtataagag acagaatgca atggatgatc tgggaaataa 60
   ga 62
<210> 96
   <211> 56
   <212> DNA
   <213> Artificial Sequence
<220>
   <223> Description of Artificial Sequence: Synthetic primer
<400> 96
   gtctcgtggg ctcggagatg tgtataagag acagtgatgc cttgacctcc tgatct 56
<210> 97
   <211> 64
   <212> DNA
   <213> Artificial Sequence
<220>
   <223> Description of Artificial Sequence: Synthetic primer
<400> 97
   gtctcgtggg ctcggagatg tgtataagag acagactaga gtgtctgtgt aatcaaacaa 60
   gttt 64
<210> 98
   <211> 52
   <212> DNA
   <213> Artificial Sequence
<220>
   <223> Description of Artificial Sequence: Synthetic primer
<400> 98
   gtctcgtggg ctcggagatg tgtataagag acagcaggcg tcctactggc at 52
<210> 99
   <211> 56
   <212> DNA
   <213> Artificial Sequence
<220>
   <223> Description of Artificial Sequence: Synthetic primer
<400> 99
   gtctcgtggg ctcggagatg tgtataagag acagctcctt caccttgccg taagag 56
<210> 100
   <211> 63
   <212> DNA
   <213> Artificial Sequence
<220>
   <223> Description of Artificial Sequence: Synthetic primer
<400> 100
   gtctcgtggg ctcggagatg tgtataagag acaggacatc tccattcttc tcttttaatt 60
   gcc 63
<210> 101
   <211> 52
   <212> DNA
   <213> Artificial Sequence
<220>
   <223> Description of Artificial Sequence: Synthetic primer
<400> 101
   gtctcgtggg ctcggagatg tgtataagag acagcctagc acgtgcctac ct 52
<210> 102
   <211> 59
   <212> DNA
   <213> Artificial Sequence
<220>
   <223> Description of Artificial Sequence: Synthetic primer
<400> 102
   gtctcgtggg ctcggagatg tgtataagag acagtggtga aacctgtttg ttggacata 59
<210> 103
   <211> 58
   <212> DNA
   <213> Artificial Sequence
<220>
   <223> Description of Artificial Sequence: Synthetic primer
<400> 103
   gtctcgtggg ctcggagatg tgtataagag acagcacagc aaagcagaaa ctcacatc 58
<210> 104
   <211> 56
   <212> DNA
   <213> Artificial Sequence
<220>
   <223> Description of Artificial Sequence: Synthetic primer
<400> 104
   gtctcgtggg ctcggagatg tgtataagag acagtgtgcc agggacctta ccttat 56
<210> 105
   <211> 55
   <212> DNA
   <213> Artificial Sequence
<220>
   <223> Description of Artificial Sequence: Synthetic primer
<400> 105
   gtctcgtggg ctcggagatg tgtataagag acagatagtc caggaggcag ccgaa 55
<210> 106
   <211> 60
   <212> DNA
   <213> Artificial **Sequence**
<220>
   <223> Description of Artificial Sequence: Synthetic primer
<400> 106
   gtctcgtggg ctcggagatg tgtataagag acagatgctg agatcagcca aattcagtta 60
<210> 107
   <211> 57
   <212> DNA
   <213> Artificial Sequence
<220>
   <223> Description of Artificial Sequence: Synthetic primer
<400> 107
   gtctcgtggg ctcggagatg tgtataagag acaggtcaag cagagaatgg gtactca 57
<210> 108
   <211> 56
   <212> DNA
   <213> Artificial Sequence
<220>
   <223> Description of Artificial Sequence: Synthetic primer
<400> 108
   gtctcgtggg ctcggagatg tgtataagag acagcaggaa aatgctggct gaccta 56
<210> 109
   <211> 58
   <212> DNA
   <213> Artificial Sequence
<220>
   <223> Description of Artificial Sequence: Synthetic primer
<400> 109
   gtctcgtggg ctcggagatg tgtataagag acagtaaggc ctgctgaaaa tgactgaa 58
<210> 110
   <211> 56
   <212> DNA
   <213> Artificial Sequence
<220>
   <223> Description of Artificial Sequence: Synthetic primer
<400> 110
   gtctcgtggg ctcggagatg tgtataagag acagaggact tgggaggtat ccacat 56
<210> 111
   <211> 64
   <212> DNA
   <213> Artificial Sequence
<220>
   <223> Description of Artificial Sequence: Synthetic primer
<400> 111
   gtctcgtggg ctcggagatg tgtataagag acagcttgct ctgataggaa aatgagatct 60
   actg 64
<210> 112
   <211> 61
   <212> DNA
   <213> Artificial Sequence
<220>
   <223> Description of Artificial Sequence: Synthetic primer
<400> 112
   gtctcgtggg ctcggagatg tgtataagag acagtgtgga agatccaatc catttttgtt 60
   g 61
<210> 113
   <211> 55
   <212> DNA
   <213> Artificial Sequence
<220>
   <223> Description of Artificial Sequence: Synthetic primer
<400> 113
   gtctcgtggg ctcggagatg tgtataagag acagtgaaag ctgtaccata cctgt 55
<210> 114
   <211> 58
   <212> DNA
   <213> Artificial Sequence
<220>
   <223> Description of Artificial Sequence: Synthetic primer
<400> 114
   gtctcgtggg ctcggagatg tgtataagag acagaggtta atatccgcaa atgacttg 58
<210> 115
   <211> 55
   <212> DNA
   <213> Artificial Sequence
<220>
   <223> Description of Artificial Sequence: Synthetic primer
<400> 115
   gtctcgtggg ctcggagatg tgtataagag acagagatga tccgacaagt gagag 55
<210> 116
   <211> 52
   <212> DNA
   <213> Artificial Sequence
<220>
   <223> Description of Artificial Sequence: Synthetic primer
<400> 116
   gtctcgtggg ctcggagatg tgtataagag acagtacgtc tcctccgacc ac 52
<210> 117
   <211> 52
   <212> DNA
   <213> Artificial Sequence
<220>
   <223> Description of Artificial Sequence: Synthetic primer
<400> 117
   gtctcgtggg ctcggagatg tgtataagag acagcgcagc ctgtacccag tg 52
<210> 118
   <211> 56
   <212> DNA
   <213> Artificial Sequence
<220>
   <223> Description of Artificial Sequence: Synthetic primer
<400> 118
   gtctcgtggg ctcggagatg tgtataagag acaggcggaa gatgaagatt tcggat 56
<210> 119
   <211> 59
   <212> DNA
   <213> Artificial Sequence
<220>
   <223> Description of Artificial Sequence: Synthetic primer
<400> 119
   gtctcgtggg ctcggagatg tgtataagag acaggttagc tcatttttgt taatggtgg 59
<210> 120
   <211> 60
   <212> DNA
   <213> Artificial Sequence
<220>
   <223> Description of Artificial Sequence: Synthetic primer
<400> 120
   gtctcgtggg ctcggagatg tgtataagag acagttaaac ttttctttta gttgtgctga 60
<210> 121
   <211> 61
   <212> DNA
   <213> Artificial Sequence
<220>
   <223> Description of Artificial Sequence: Synthetic primer
<400> 121
   gtctcgtggg ctcggagatg tgtataagag acaggtatgc aacatttcta aagttaccta 60
   c 61
<210> 122
   <211> 54
   <212> DNA
   <213> Artificial Sequence
<220>
   <223> Description of Artificial Sequence: Synthetic primer
<400> 122
   gtctcgtggg ctcggagatg tgtataagag acagaagacc ataacccacc acag 54
<210> 123
   <211> 54
   <212> DNA
   <213> Artificial Sequence
<220>
   <223> Description of Artificial Sequence: Synthetic primer
<400> 123
   gtctcgtggg ctcggagatg tgtataagag acagctggaa agggacgaac tggt 54
<210> 124
   <211> 54
   <212> DNA
   <213> Artificial Sequence
<220>
   <223> Description of Artificial Sequence: Synthetic primer
<400> 124
   gtctcgtggg ctcggagatg tgtataagag acagcgaccc agttaccata gcaa 54
<210> 125
   <211> 60
   <212> DNA
   <213> Artificial Sequence
<220>
   <223> Description of Artificial Sequence: Synthetic primer
<400> 125
   gtctcgtggg ctcggagatg tgtataagag acagagaaaa tggaagtcta tgtgatcaag 60
<210> 126
   <211> 63
   <212> DNA
   <213> Artificial Sequence
<220>
   <223> Description of Artificial Sequence: Synthetic primer
<400> 126
   gtctcgtggg ctcggagatg tgtataagag acagcatttt aaattttctt tctctaggtg 60
   aag 63
<210> 127
   <211> 52
   <212> DNA
   <213> Artificial Sequence
<220>
   <223> Description of Artificial Sequence: Synthetic primer
<400> 127
   gtctcgtggg ctcggagatg tgtataagag acagggtctt ggccgaggtc tc 52
<210> 128
   <211> 52
   <212> DNA
   <213> Artificial Sequence
<220>
   <223> Description of Artificial Sequence: Synthetic primer
<400> 128
   gtctcgtggg ctcggagatg tgtataagag acagcagcac catgggcacg tc 52
<210> 129
   <211> 53
   <212> DNA
   <213> Artificial Sequence
<220>
   <223> Description of Artificial Sequence: Synthetic primer
<400> 129
   gtctcgtggg ctcggagatg tgtataagag acaggagagg tggaaagcga gag 53
<210> 130
   <211> 52
   <212> DNA
   <213> Artificial Sequence
<220>
   <223> Description of Artificial Sequence: Synthetic primer
<400> 130
   gtctcgtggg ctcggagatg tgtataagag acagcactct tgcccacacc gc 52
<210> 131
   <211> 62
   <212> DNA
   <213> Artificial Sequence
<220>
   <223> Description of Artificial Sequence: Synthetic primer
<400> 131
   gtctcgtggg ctcggagatg tgtataagag acagctgtat ttatttcagt gttacttacc 60
   tg 62
<210> 132
   <211> 64
   <212> DNA
   <213> Artificial Sequence
<220>
   <223> Description of Artificial Sequence: Synthetic primer
<400> 132
   gtctcgtggg ctcggagatg tgtataagag acagttatat tcaatttaaa cccacctata 60
   atgg 64
<210> 133
   <211> 56
   <212> DNA
   <213> Artificial Sequence
<220>
   <223> Description of Artificial Sequence: Synthetic primer
<400> 133
   gtctcgtggg ctcggagatg tgtataagag acaggtatca aagaatggtc ctgcac 56
<210> 134
   <211> 57
   <212> DNA
   <213> Artificial Sequence
<220>
   <223> Description of Artificial Sequence: Synthetic primer
<400> 134
   gtctcgtggg ctcggagatg tgtataagag acagacacaa cacaaaatag ccgtata 57
<210> 135
   <211> 59
   <212> DNA
   <213> Artificial Sequence
<220>
   <223> Description of Artificial Sequence: Synthetic primer
<400> 135
   gtctcgtggg ctcggagatg tgtataagag acagtctatt ctttcctttg tagtgtcca 59
<210> 136
   <211> 61
   <212> DNA
   <213> Artificial Sequence
<220>
   <223> Description of Artificial Sequence: Synthetic primer
<400> 136
   gtctcgtggg ctcggagatg tgtataagag acagtgattt ctgtttttac ctcctaaaga 60
   a 61
<210> 137
   <211> 64
   <212> DNA
   <213> Artificial Sequence
<220>
   <223> Description of Artificial Sequence: Synthetic primer
<400> 137
   gtctcgtggg ctcggagatg tgtataagag acagatcttt aaagagaaat ttgctaaagc 60
   tgtg 64
<210> 138
   <211> 57
   <212> DNA
   <213> Artificial Sequence
<220>
   <223> Description of Artificial Sequence: Synthetic primer
<400> 138
   gtctcgtggg ctcggagatg tgtataagag acagacgtgt tttgatcaaa gaagagg 57
<210> 139
   <211> 52
   <212> DNA
   <213> Artificial Sequence
<220>
   <223> Description of Artificial Sequence: Synthetic primer
<400> 139
   gtctcgtggg ctcggagatg tgtataagag acagagcctc acgttggtcc ac 52
<210> 140
   <211> 52
   <212> DNA
   <213> Artificial Sequence
<220>
   <223> Description of Artificial Sequence: Synthetic primer
<400> 140
   gtctcgtggg ctcggagatg tgtataagag acagggatgg ctaggcgagg ag 52
<210> 141
   <211> 52
   <212> DNA
   <213> Artificial Sequence
<220>
   <223> Description of Artificial Sequence: Synthetic primer
<400> 141
   gtctcgtggg ctcggagatg tgtataagag acagagggac taggcgtggg at 52
<210> 142
   <211> 52
   <212> DNA
   <213> Artificial Sequence
<220>
   <223> Description of Artificial Sequence: Synthetic primer
<400> 142
   gtctcgtggg ctcggagatg tgtataagag acagccaagc cctagggtgg tg 52
<210> 143
   <211> 55
   <212> DNA
   <213> Artificial Sequence
<220>
   <223> Description of Artificial Sequence: Synthetic primer
<400> 143
   gtctcgtggg ctcggagatg tgtataagag acagacgctc ttctcactca tatcc 55
<210> 144
   <211> 59
   <212> DNA
   <213> Artificial Sequence
<220>
   <223> Description of Artificial Sequence: Synthetic primer
<400> 144
   gtctcgtggg ctcggagatg tgtataagag acagaagaaa tcttagacgt aagcccctc 59
<210> 145
   <211> 52
   <212> DNA
   <213> Artificial Sequence
<220>
   <223> Description of Artificial Sequence: Synthetic primer
<400> 145
   gtctcgtggg ctcggagatg tgtataagag acagtcccat accctctcag cg 52
<210> 146
   <211> 54
   <212> DNA
   <213> Artificial Sequence
<220>
   <223> Description of Artificial Sequence: Synthetic primer
<400> 146
   gtctcgtggg ctcggagatg tgtataagag acagggatga gctacctgga ggat 54
<210> 147
   <211> 58
   <212> DNA
   <213> Artificial Sequence
<220>
   <223> Description of Artificial Sequence: Synthetic primer
<400> 147
   gtctcgtggg ctcggagatg tgtataagag acagattttg agtgttagac tggaaact 58
<210> 148
   <211> 53
   <212> DNA
   <213> Artificial Sequence
<220>
   <223> Description of Artificial Sequence: Synthetic primer
<400> 148
   gtctcgtggg ctcggagatg tgtataagag acaggggcag tgctaggaaa gag 53
<210> 149
   <211> 53
   <212> DNA
   <213> Artificial Sequence
<220>
   <223> Description of Artificial Sequence: Synthetic primer
<400> 149
   gtctcgtggg ctcggagatg tgtataagag acagtgctta cctcgcttag tgc 53
<210> 150
   <211> 52
   <212> DNA
   <213> Artificial Sequence
<220>
   <223> Description of Artificial Sequence: Synthetic primer
<400> 150
   gtctcgtggg ctcggagatg tgtataagag acaggccggg gatgtgatga ga 52
<210> 151
   <211> 52
   <212> DNA
   <213> Artificial Sequence
<220>
   <223> Description of Artificial Sequence: Synthetic primer
<400> 151
   gtctcgtggg ctcggagatg tgtataagag acagcagggg tcagaggcaa gc 52
<210> 152
   <211> 52
   <212> DNA
   <213> Artificial Sequence
<220>
   <223> Description of Artificial Sequence: Synthetic primer
<400> 152
   gtctcgtggg ctcggagatg tgtataagag acagagggcc actgacaacc ac 52

## Description

### BACKGROUND OF THE DISCLOSURE

The disclosure relates, in general, to targeted enrichment of nucleic acids and, more particularly, to a system and method for transposase-mediated fragmentation and amplification-based enrichment with unidirectional sequence-specific primers.

Whole genome sequencing is a valuable tool for both research and clinical applications. For example, sequencing can provide a comprehensive view of the entire genome and allow for the detection of single nucleotide variants, nucleotide insertions and deletions, and large structural variants. However, sequencing entire genomes can be costly, and researchers and clinicians may only be interested in genetic information from particular regions of interest. In these cases, target enrichment ahead of sequencing is a more attractive option.

Targeted enrichment methods for sequencing can be broadly divided into two categories: i) hybridization-based capture methods, and ii) polymerase chain reaction (PCR)-based (i.e., amplification-based) enrichment methods. For many applications, hybridization methods can be more sensitive methods for identifying single nucleotide polymorphisms (SNPs) at low minor allele frequencies, but may suffer from a need for large starting material input requirements (e.g., more than 100 ng of DNA or RNA), laborious workflows (e.g., time intensive, extensive hands-on time), and high costs. PCR methods are generally less sensitive with respect to SNP detection as the priming sites are always the same for each of the different templates in a sample, which means that PCR duplicates cannot be identified. Novel gene fusion events can also not be identified by standard amplification-based methods, because only targets containing primer binding sites for both forward and reverse primers are amplified and subsequently sequenced.

Accordingly, there is a need for improved processes and systems for targeted enrichment for sequencing. In this context, WO2016/033251 and WO2016/061517 already disclose enrichment of a particular target region

### SUMMARY OF THE DISCLOSURE

The present invention overcomes the aforementioned drawbacks by providing a system and method for transposase-mediated amplification-based sequencing.

In accordance with one aspect of the present disclosure, method for targeted enrichment of nucleic acids includes contacting a nucleic acid including at least one region of interest with a plurality of transposase complexes. Each of the transposase complexes includes at least a transposase and a first polynucleotide having a transposon end sequence and a first label sequence. The method further includes incubating the nucleic acid and the transposase complexes under conditions whereby the nucleic acid is fragmented into a plurality of nucleic acid fragments including first polynucleotide attached to each 5' end of the nucleic acid fragments. The method further includes selectively amplifying the nucleic acid fragments, thereby enriching for a portion of the nucleic acid fragments including the at least one region of interest relative to a remaining portion of the nucleic acid fragments, and sequencing the enriched nucleic acid fragments. In certain embodiments of the methods of the disclosure, the amplifying step comprises a denaturation step. In certain embodiments of the methods of the disclosure, the amplifying step does not comprise a denaturation step and the amplifying step comprises a nick-translation step. By inclusion of the nick-translation step, the amplifying step produces a plurality of nucleic acid fragments including the first polynucleotide attached to each 5' end of the nucleic acid fragments and the complement of the first polynucleotide attached to each 3' end of the nucleic acid fragments.

The foregoing and other aspects and advantages of the invention will appear from the following description. In the description, reference is made to the accompanying drawings which form a part hereof, and in which there is shown by way of illustration a preferred embodiment of the invention. Such embodiment does not necessarily represent the full scope of the invention, however, and reference is made therefore to the claims and herein for interpreting the scope of the invention.

### BRIEF DESCRIPTION OF THE DRAWINGS

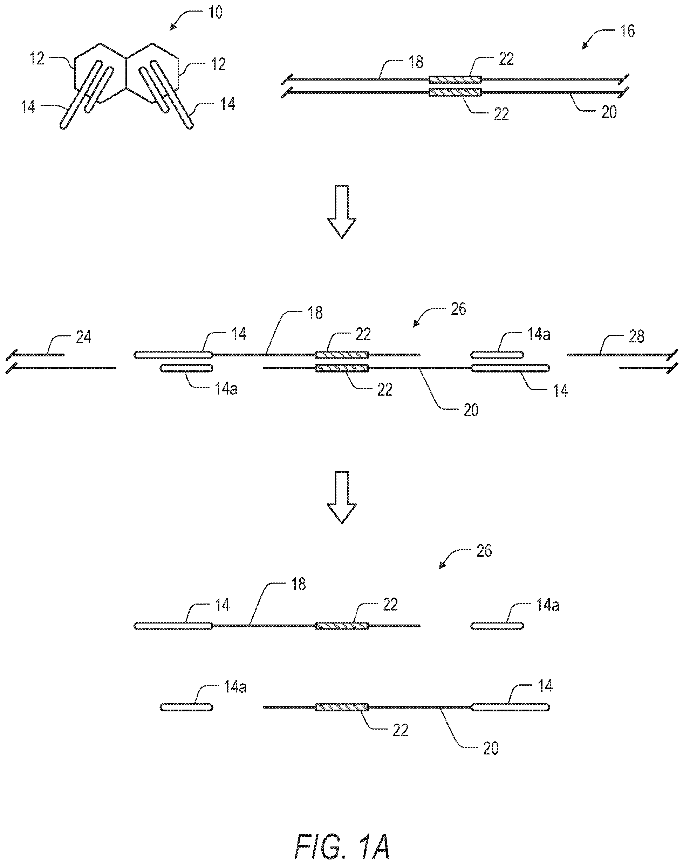

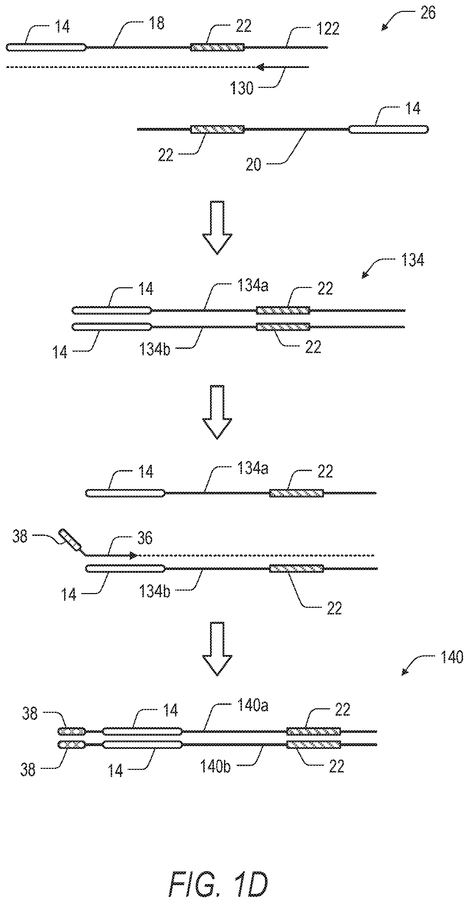

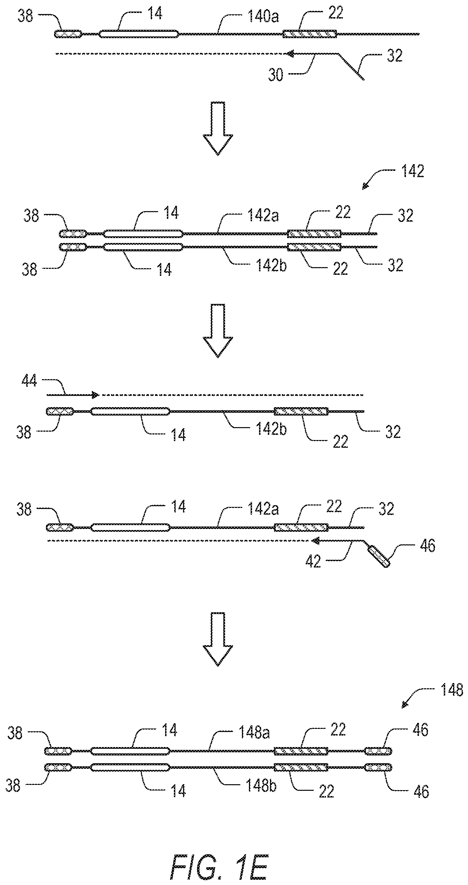

Fig. 1A is a schematic illustration showing a first phase in a method for transposase-mediated amplicon sequencing according to the present disclosure. In the first phase of the method, a transposase complex is formed using common end sequences, and the complex is exposed to a nucleic acid sample. Following transposase-based fragmentation and labeling of the nucleic acid sample, the fragmented nucleic acids are denatured in preparation for a second phase of the method.

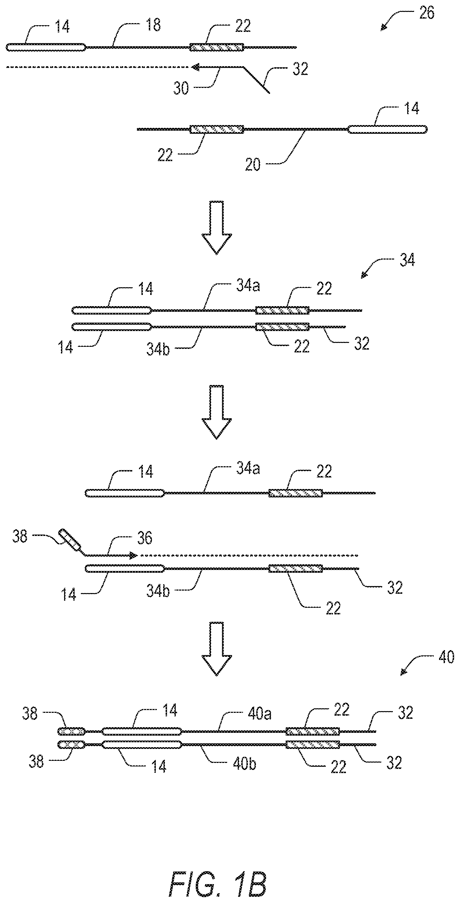

Fig. 1B is a schematic illustration showing a second phase in the method of Fig. 1A. In the second phase of the method, the fragmented and denatured nucleic acids are amplified using sequence specific primers paired with adapter specific primers for amplification-based enrichment of one or more regions of interest. Dotted lines extending from primers indicate extension of the primer and replication of the template strand by a polymerase.

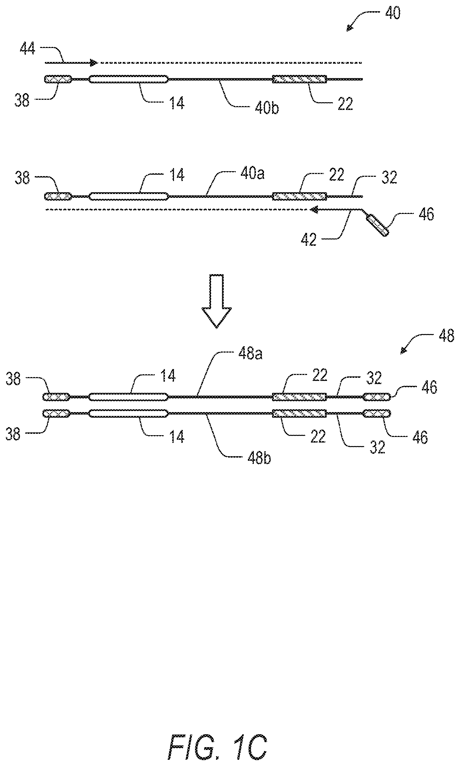

Fig. 1C is a schematic illustration showing a third phase of the method of Figs. 1A and 1B. In the third phase of the method, a second primer set is used to further amplify and tag the amplicons generated in the second phase of the method in preparation for sequencing. Dotted lines extending from primers indicate extension of the primer and replication of the template strand by a polymerase.
Fig. ID is a schematic illustration showing an alternative embodiment of a second phase in the method of Fig. 1A. In the second phase of the method, the fragmented and denatured nucleic acids are amplified using out-nested sequence specific primers paired with adapter specific primers for amplification-based enrichment of one or more regions of interest. Dotted lines extending from primers indicate extension of the primer and replication of the template strand by a polymerase.
Fig. IE is a schematic illustration showing a third phase of the method of Figs. 1A and 1D, including a second round of amplification with in-nested target specific primers. In the third phase of the method, a second primer set is used to further amplify and tag the amplicons generated in the second phase of the method in preparation for sequencing. Dotted lines extending from primers indicate extension of the primer and replication of the template strand by a polymerase.
Fig. 2A is schematic illustration of an example method for targeted enrichment of ribonucleic acids (i.e., RNA) including cDNA synthesis, end repair and dA-tailing, adapter ligation, and PCR.
Fig. 2B is a schematic illustration of a method according to the present disclosure for targeted enrichment of RNA including transposase based fragmentation and labeling of nucleic acids, followed by PCR. Notably, the method of Fig. 2B eliminates a number of steps present in the method of Fig. 2A.
Fig. 3A is a schematic illustration of another example method for targeted enrichment of deoxyribonucleic acids (i.e., DNA) including fragmentation, end repair and dA-tailing, adapter ligation, and PCR.
Fig. 3B is a schematic illustration of a method according to the present disclosure for targeted enrichment of DNA including transposase based fragmentation and labeling of nucleic acids, followed by PCR. Notably, the method of Fig. 3B eliminates a number of steps present in the method of Fig. 3A.

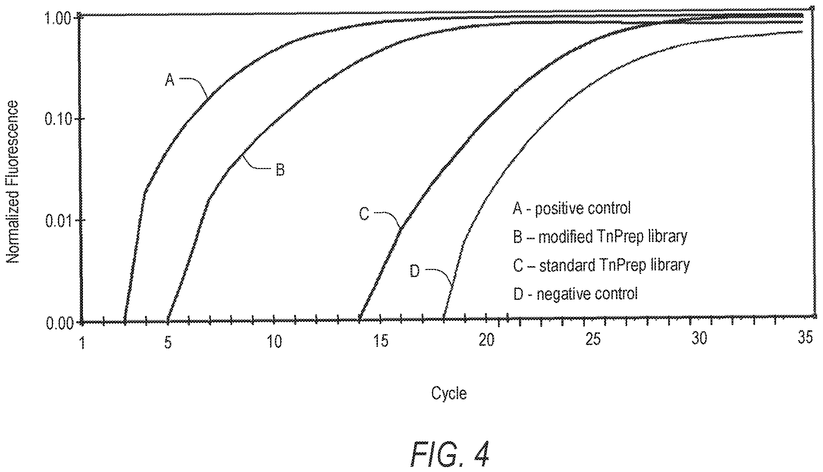

Fig. 4 is a plot of real-time PCR data showing normalized fluorescence signal as a function of cycle number for data collected according to an embodiment of the system and method of the present disclosure. Real-time PCR data was collected for four different samples (curves labeled A-D): Curve A - positive control (unsheared DNA pre-amplified with gene specific primers); Curve B - modified TnPrep library (TnPrep with gene specific primers for targeted amplification using a 2-step PCR protocol); Curve C - standard TnPrep library (TnPrep with non-specific amplification); Curve D - negative control (unsheared DNA). Data generated using primers specific to regions of interest showed approximately 1000-fold enrichment for region of interest in the modified TnPrep library (curve B) compared to the standard TnPrep library (curve C). The "standard TnPrep library" is a library prepared with the standard transposase loaded with R1 and R2 arms and amplified with the standard NEXTERA i5 and i7 primers (ILLUMINA, INC.), as opposed to the "modified TnPrep library" which is prepared with a transposase loaded with only R1 arms and amplified with NEXTERA i5 and target specific primers.
Fig. 5 is a nucleic acid analysis trace showing fluorescence signal as a function of nucleic acid size (bp) for sequencing libraries prepared according to the methods of the present disclosure (including a 2-step PCR protocol), and with higher concentrations of transposase (36 µg/ml). Data is shown for i) sequencing libraries prepared with sequence specific primers to enrich for target regions of interest, and ii) standard sequencing libraries prepared without specific primers.
Fig. 6 is a nucleic acid analysis trace showing fluorescence signal as a function of nucleic acid size (bp) for sequencing libraries prepared according to the methods of the present disclosure (using a 2-step PCR protocol), and with lower concentrations of transposase (4 µg/ml). Data is shown for i) sequencing libraries prepared with sequence specific primers to enrich for target regions of interest, and ii) standard sequencing libraries prepared with non-specific primers.

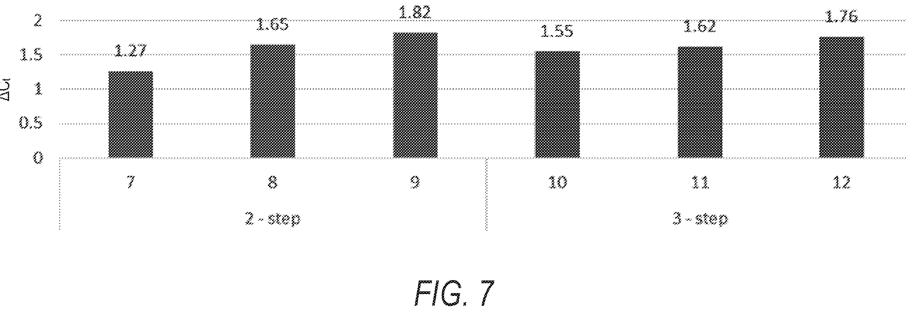

Fig. 7 is a bar chart illustrating the specificity of amplification assessed by real-time PCR using primers specific to the adapter sequences, and in separate reactions, primers specific to the target regions. In the situation where all fragments with adapter sequences on both ends contain the region of interest, the difference between the two Cₜ (i.e., the threshold cycle) values is expected to be minimal. For non-specific amplification, fragments can result that have both adapter sequences, but do not contain the region of interest, and in this case the difference between the Cₜ values would be larger. The bar chart illustrates that for both the 2-step and 3-step amplification/PCR workflows/protocols the delta Cₜ is low, indicating specific amplification of regions of interest. Labels on the horizontal axis indicate results for libraries prepared with different cycle numbers as follows (notation is in the format: [sample #] - [# of cycles in 1^{st} round of PCR]/[# of cycles in 2^{nd} round of PCR]/[# of cycles in 3^{rd} round of PCR (if applicable)]): 7 - 20/12; 8 - 15/15; 9 - 10/20; 10 - 20/8/8; 11 - 15/10/7; 12 - 10/10/12.
Fig. 8 is a bar chart illustrating percent on target rate as determined by sequencing. Highly specific amplification and successful enrichment of regions of interest, with greater than 90% on target rate was achieved in all cases. Labels on the horizontal axis indicate results for libraries prepared with different cycle numbers as follows (notation is in the format: [sample #] - [# of cycles in 1^{st} round of PCR]/[# of cycles in 2^{nd} round of PCR]/[# of cycles in 3^{rd} round of PCR (if applicable)]): 7 - 20/12; 8 - 15/15; 9 - 10/20; 10 - 20/8/8; 11 - 15/10/7; 12 - 10/10/12.
Fig. 9 is a bar graph showing the effect of transposase complex concentration on the percentage of on-target reads.
Fig. 10A is a bar graph showing the effect of DNA input on the percentage of on-target reads.
Fig. 10B is plot of normalized coverage as a function of target DNA amplicon showing the effect of DNA input on coverage uniformity.
Fig. 11 is a parity plot comparing observed and expected alternate allele frequencies for a library prepared from 50 ng of a reference standard including known variants. Error bars are indicative of the standard deviation from 3 technical replicates.
Fig. 12 is a bar graph illustrating the on-target rates of libraries constructed from different FFPET DNA samples according to the present disclosure.
Fig. 13 is a bar graph illustrating the on-target rates of libraries constructed from reactions wherein the heat denaturation step is eliminated and wherein a nick-translation step has been added. The results of the nick-translation step were compared to a heat-treated condition. The nick-translation step was included to ensure that an adapter would be present on both 5' and 3' ends.
Fig. 14A is a bar graph illustrating quality control of fragments prior to sequencing. Similar to Fig. 13, some reactions included a heat treatment step (HT) while others included a nick-translation step (NT). The R1 arms used in these reactions include molecular barcodes (also referred to herein as unique molecular identifiers (UIDs)) in place of i5 index (See Example 6).
Fig. 14B is a series of graphs illustrating the use of standard versus UID-containing R1 arms in either a 31-primer or a 40-primer panel.

The Like numbers will be used to describe like parts from Figure to Figure throughout the following detailed description.

### DETAILED DESCRIPTION OF THE DISCLOSURE

### 1. Definitions

The terms "a", "an" and "the" generally include plural referents, unless the context clearly indicates otherwise.

The term "amplification" generally refers to the production of a plurality of nucleic acid molecules from a target nucleic acid wherein primers hybridize to specific sites on the target nucleic acid molecules in order to provide an initiation site for extension by a polymerase. Amplification can be carried out by any method generally known in the art, such as but not limited to: standard PCR, long PCR, hot start PCR, qPCR, RT-PCR and Isothermal Amplification. Other amplification reactions comprise, among others, the Ligase Chain Reaction, Polymerase Ligase Chain Reaction, Gap-LCR, Repair Chain Reaction, 3SR, NASBA, Strand Displacement Amplification (SDA), Transcription Mediated Amplification (TMA), and Qb-amplification.

The term "complementary" generally refers to the ability to form favorable thermodynamic stability and specific pairing between the bases of two nucleotides at an appropriate temperature and ionic buffer conditions. This pairing is dependent on the hydrogen bonding properties of each nucleotide. The most fundamental examples of this are the hydrogen bond pairs between thymine/adenine and cytosine/guanine bases. In the present invention, primers for amplification of target nucleic acids can be both fully complementary over their entire length with a target nucleic acid molecule and "semi-complementary" wherein the primer contains additional, non-complementary sequence minimally capable or incapable of hybridization to the target nucleic acid.

The term "detecting" as used herein relates to a qualitative test aimed at assessing the presence or absence of a target nucleic acid in a sample.

The term "enriched" as used herein relates to any method of treating a sample comprising a target nucleic acid that allows for separating the target nucleic acid from at least a part of other material present in the sample. "Enrichment" can, thus, be understood as a production of a higher amount of target nucleic acid over other material.

The term "excess" generally refers to a larger quantity or concentration of a certain reagent or reagents as compared to another.

The term "hybridize" generally refers to the base-pairing between different nucleic acid molecules consistent with their nucleotide sequences. The terms "hybridize" and "anneal" can be used interchangeably.

The terms "nucleic acid" or "polynucleotide" can be used interchangeably and refer to a polymer that can be corresponded to a ribose nucleic acid (RNA) or deoxyribose nucleic acid (DNA) polymer, or an analog thereof. This includes polymers of nucleotides such as RNA and DNA, as well as synthetic forms, modified (e.g., chemically or biochemically modified) forms thereof, and mixed polymers (e.g., including both RNA and DNA subunits). Exemplary modifications include methylation, substitution of one or more of the naturally occurring nucleotides with an analog, internucleotide modifications such as uncharged linkages (e.g., methyl phosphonates, phosphotriesters, phosphoamidates, carbamates, and the like), pendent moieties (e.g., polypeptides), intercalators (e.g., acridine, psoralen, and the like), chelators, alkylators, and modified linkages (e.g., alpha anomeric nucleic acids and the like). Also included are synthetic molecules that mimic polynucleotides in their ability to bind to a designated sequence via hydrogen bonding and other chemical interactions. Typically, the nucleotide monomers are linked via phosphodiester bonds, although synthetic forms of nucleic acids can comprise other linkages (e.g., peptide nucleic acids as described in Nielsen et al. (Science 254:1497-1500, 1991). A nucleic acid can be or can include, e.g., a chromosome or chromosomal segment, a vector (e.g., an expression vector), an expression cassette, a naked DNA or RNA polymer, the product of a polymerase chain reaction (PCR), an oligonucleotide, a probe, and a primer. A nucleic acid can be, e.g., single-stranded, double-stranded, or triple-stranded and is not limited to any particular length. Unless otherwise indicated, a particular nucleic acid sequence comprises or encodes complementary sequences, in addition to any sequence explicitly indicated.

The term "nucleotide" in addition to referring to the naturally occurring ribonucleotide or deoxyribonucleotide monomers, shall herein be understood to refer to related structural variants thereof, including derivatives and analogs, that are functionally equivalent with respect to the particular context in which the nucleotide is being used (e.g., hybridization to a complementary base), unless the context clearly indicates otherwise.

The term "oligonucleotide" refers to a nucleic acid that includes at least two nucleic acid monomer units (e.g., nucleotides). An oligonucleotide typically includes from about six to about 175 nucleic acid monomer units, more typically from about eight to about 100 nucleic acid monomer units, and still more typically from about 10 to about 50 nucleic acid monomer units (e.g., about 15, about 20, about 25, about 30, about 35, or more nucleic acid monomer units). The exact size of an oligonucleotide will depend on many factors, including the ultimate function or use of the oligonucleotide. Oligonucleotides are optionally prepared by any suitable method, including, but not limited to, isolation of an existing or natural sequence, DNA replication or amplification, reverse transcription, cloning and restriction digestion of appropriate sequences, or direct chemical synthesis by a method such as the phosphotriester method of Narang et al. (Meth. Enzymol. 68:90-99, 1979); the phosphodiester method of Brown et al. (Meth. Enzymol. 68:109-151, 1979); the diethylphosphoramidite method of Beaucage et al. (Tetrahedron Lett. 22:1859-1862, 1981); the triester method of Matteucci et al. (J. Am. Chem. Soc. 103:3185-3191, 1981); automated synthesis methods; Maskless Array Synthesis as disclosed in Singh-Gasson et al., Nature Biotechnology, 17: 974-978, 1999, or the solid support method of U.S. Pat. No. 4,458,066, or other methods known to those skilled in the art.

The term "primer" refers to a polynucleotide capable of acting as a point of initiation of template-directed nucleic acid synthesis when placed under conditions in which polynucleotide extension is initiated (e.g., under conditions comprising the presence of requisite nucleoside triphosphates (as dictated by the template that is copied) and a polymerase in an appropriate buffer and at a suitable temperature or cycle(s) of temperatures (e.g., as in a polymerase chain reaction)). To further illustrate, primers can also be used in a variety of other oligonucleotide-mediated synthesis processes, including as initiators of de novo RNA synthesis and in vitro transcription-related processes (e.g., nucleic acid sequence-based amplification (NASBA), transcription mediated amplification (TMA), etc.). A primer is typically a single-stranded oligonucleotide (e.g., oligodeoxyribonucleotide). The appropriate length of a primer depends on the intended use of the primer but typically ranges from 6 to 40 nucleotides, more typically from 15 to 35 nucleotides. Short primer molecules generally require cooler temperatures to form sufficiently stable hybrid complexes with the template. A primer need not reflect the exact sequence of the template but must be sufficiently complementary to hybridize with a template for primer elongation to occur. In certain embodiments, the term "primer pair" means a set of primers including a 5' sense primer (sometimes called "forward") that hybridizes with the complement of the 5' end of the nucleic acid sequence to be amplified and a 3' antisense primer (sometimes called "reverse") that hybridizes with the 3' end of the sequence to be amplified (e.g., if the target sequence is expressed as RNA or is an RNA). A primer can be labeled, if desired, by incorporating a label detectable by spectroscopic, photochemical, biochemical, immunochemical, or chemical means. For example, useful labels include 32P, fluorescent dyes, electron-dense reagents, enzymes (as commonly used in ELISA assays), biotin, or haptens and proteins for which antisera or monoclonal antibodies are available.

In the sense of the invention, "purification", "isolation" or "extraction" of nucleic acids relate to the following: Before nucleic acids may be analyzed in a diagnostic assay e.g. by amplification, they typically have to be purified, isolated or extracted from biological samples containing complex mixtures of different components. For the first steps, processes may be used which allow the enrichment of the nucleic acids. Such methods of enrichment are described herein.

The term "quantitating" as used herein relates to the determination of the amount or concentration of a target nucleic acid present in a sample.

"Target nucleic acid" is used herein to denote a nucleic acid in a sample which should be analyzed, i.e. the presence, non-presence, nucleic acid sequence and/or amount thereof in a sample should be determined. The target nucleic acid may be a genomic sequence, e.g. part of a specific gene, RNA, cDNA or any other form of nucleic acid sequence. In some embodiments, the target nucleic acid may be viral or microbial.

The terms "target nucleic acid", and "target molecule" can be used interchangeably and refer to a nucleic acid molecule that is the subject of an amplification reaction that may optionally be interrogated by a sequencing reaction in order to derive its sequence information.

The terms "target specific region" or "region of interest" can be used interchangeably and refer to the region of a particular nucleic acid molecule that is of scientific interest. These regions typically have at least partially known sequences in order to design primers which flank the region or regions of interest for use in amplification reactions and thereby recover target nucleic acid amplicons containing these regions of interest.

The term "maskless array synthesis" (MAS) refers to light-directed synthesis of oligonucleotides on the surface of a substrate as an array in the absence of a physical mask, such as the method as described by Singh-Gasson et al., Nature Biotech, 17: 974-978 (October 1999), the teachings of which are hereby incorporated by reference. Briefly, the MAS technique generally uses a digital microarray mirror device (DMD) which consists of micromirrors to form virtual masks. These mirrors are individually addressable and can be used to create any given pattern or image in a broad range of wavelengths. The DMD forms an image on the surface of the substrate, wherein the substrate contains chemical moieties that are activated by light. A solution containing a given nucleotide is then washed over the surface of the substrate, and binds to the activated regions. The nucleotide in the solution contains are photoprotected with a protecting group that is photolabile. In a second round of synthesis, the DMD forms a second image onto selected regions of the substrate, thereby selectively activating the substrate in those regions, and a second given nucleotide (again, photoprotected) is washed over the substrate. This second nucleotide binds to those regions that have been activated during the second round of illumination. Thus, selected nucleotides can be added to selected regions, allowing for synthesis of an array of oligonucleotides through light-directed synthesis in the absence of a mask. This process is repeated numerous times in order to build the oligonucleotides sequences on a monomer-by-monomer basis.

Other methods of building arrays can also be used in the present invention, such as the use of chromium masks or spotting of oligonucleotides on an array. MAS provides improved flexibility and simplicity when used in the present invention, but other means of forming arrays are useful as well. Examples of the synthetic systems, besides MAS, that can be used in the present invention are those well-known methods used by Affymetrix, Oxford Gene Technologies, and Agilent.

### 2. Description

The first clinically relevant gene fusion (BCR-Abl) was identified in 1960, and is formed as a result of a translocation between chromosomes 9 and 22. The resulting fusion protein, an unregulated mutant tyrosine kinase, leads to uncontrolled cell growth and the development of the blood cancer, chronic myelogenous leukemia. Based on an understanding of the fusion product, a targeted therapy could be developed that makes use of tyrosine kinase inhibitors. In recent years, fusion genes have also been identified in numerous solid tumor cancers including colorectal, lung, prostate, breast and stomach. The abnormal protein products of fusion genes are active only in the cancer cells, and are thus potentially good targets for drug intervention with minimal toxic side effects.

Existing target enrichment methods capable of detecting novel gene fusions include the Archer Fusion Plex kit (Archer Dx) which requires cDNA synthesis followed by end repair, dA-tailing and adapter ligation followed by two PCR steps. Similarly, NuGEN's Single Primer Enrichment Technology also requires separate steps for fragmentation, end repair and ligation of adapters, prior to annealing and extension of target specific probes and a subsequent PCR step.

In general, the present disclosure provides for a combination of "transposase-based library preparation" (hereinafter, "TnPrep"), and multiplexed amplicon sequencing. In one embodiment the present disclosure provides for a method combining a modified TnPrep approach with one or more primers targeting a specific region of interest for targeted sequencing. In one aspect, embodiments of TnPrep can employ a transposition reaction to simultaneously fragment and tag DNA. Examples of tags that can be appended during the fragmentation process include nucleic acid sequence tags for a given sequencing platform, unique barcodes sequences, sample index sequences, the like, and combinations thereof.

The proposed invention has a simple and relatively fast workflow including fragmentation and simultaneous addition of sequencing adapters to the 5'-ends of the fragmented target DNA (TnPrep) by incubating target DNA with a transposase enzyme containing arms with a portion of the adapter sequences required for platform specific sequencing (e.g., ILLUMINA sequencing). The reaction product can be treated to ensure that no nick translation takes place in the subsequent amplification steps, and thus fragments only contain a single adapter sequence at the 5'-ends. PCR amplification can be performed with standard indexing primers to complete the adapter sequence and add any necessary indices, as well as a primer (or primers) targeting the region(s) of interest (ROI). In one example, a first round of PCR with target-specific primers is performed, followed by a second PCR with in-nested target specific primers that can include an out-nested portion containing part of the adapter sequence. Thereafter, a final PCR step adds in the sequencing index, producing libraries that are ready for sequencing. It is also possible to combine the second and final PCR steps into one reaction.

Notably, the present disclosure demonstrated a number of advantages over alternative approaches to targeted enrichment for library preparation. In one aspect, the low start site complexity and inability to identify duplicates inherent in traditional PCR-based (i.e., amplicon) sequencing methods is addressed by the present disclosure as TnPrep generates a diversity of start sites and offers the opportunity to introduce unique molecular identifiers (UIDs) to each nucleic acid fragment. In another aspect, embodiments of the present disclosure provides for the identification of novel gene fusion events, as there is only a single target-specific primer. Accordingly, prior knowledge of the fusion partner or breakpoint is not required. In yet another aspect, the workflows described herein are straightforward and relatively fast in comparison with alternative approaches. In yet a further aspect, embodiments of the present disclosure are applicable to a variety of starting materials including both RNA and DNA.

Turning now to Figs. 1A to 1C a one method of target enrichment including a 2-step PCR workflow for target enrichment includes preparation of a transposase complex 10. The transposase complex includes a pair of transposase enzymes 12, where each of the transposase enzymes includes a first polynucleotide 14 having a transposon end sequence and a label sequence. Notably, the transposon end sequence corresponds to the first transposase. In one aspect, the transposase is a hyperactive transposase such as hyperactive Tn5. In another aspect the transposase is wild-type or mutant transposase derived from another source organism, such as *Alishewanella aestuarii*, *Vibrio cholerae*, and *Vibrio harveyi.* In yet another aspect, another like transposase enzyme may be used.

The transposase complex 10 is incubated with a nucleic acid 16, such as a genomic DNA fragment. The nucleic acid 16 includes a top strand 18 and a bottom strand 20, as well as a target region or region of interest (ROI) 22. Following incubation, the transposase complex 10 concomitantly fragments the nucleic acid 16 into a plurality of fragments including the fragment 24, the fragment 26, and the fragment 28. The fragment 26 includes the ROI 22, and further includes the polynucleotides 14 attached to the 5' ends of the top strand 18 and bottom strand 20 of the fragment 26.

In one aspect, the fragment 26 can be denatured without the need for a gap fill reaction and ligation or PCR extension step to further prepare the fragments as in other TnPrep methods described elsewhere. Accordingly, when the fragment 26 is denatured, the portions 14a of the polynucleotides 14 can become dissociated from top strand 18 and bottom strand 20.

Turning to Fig. 1B, a first round of PCR amplification can be performed on the top strand 18 and bottom strand 20 of the fragment 26. A region specific primer 30 including a tail sequence 32 is complementary to the ROI 22, and can therefore be used to selectively enrich for the ROI 22. In one aspect, the region specific primer 30 can be used to target only one strand (e.g., top strand 18) of the (denatured) fragment 26.

The amplification product 34 (including top strand 34a and bottom strand 34b) following the first round extension with the primer 32 can be denatured to provide a template strand (i.e., bottom strand 34b) for a label specific primer 36 having a tail sequence 38. Notably, ROI 22 can only be exponentially amplified if the fragment 26 includes the ROI 22 proximal to the polynucleotide 14 to provide the amplification product 40 (including top strand 40a and bottom strand 40b).

Turning to Fig. 1C, The amplification product 40 is further amplified in a second round of PCR using a first primer 42 complementary to at least a portion of the tail sequence 32 added by the region specific primer 30, and second primer 44 complementary to at least a portion of the sequence 38 added by the label specific primer 36. The first primer 42 can also include a tail sequence 46. In one aspect, the tail sequence 38 and the tail sequence 46 can include labels, adapters, or indexes for platform specific sequencing. The resulting enriched amplification product 48 (including top strand 48a and bottom strand 48b) can then be sequenced.

Turning now to Figs. ID and IE, another method of target enrichment according to the present disclosure can include a 3-step PCR workflow. In the 3-step workflow, the fragment 26 is prepared as previously described and illustrated with respect to Fig. 1A. However, in contrast to the 2-step PCR workflow illustrated in Figs. 1B and 1C, the 3-step PCR workflow includes a first out-nested round of PCR step followed by a second in-nested round of PCR, and a third and final round of PCR to add terminal adapter sequence(s). The second and third rounds of PCR are comparable to the workflow illustrated in Figs. 1B and 1C. In one aspect, it can be useful to employ a nested PCR strategy to improve specificity and achieve a higher on-target rate. However, in some embodiments, the second and third rounds of PCR can be combined into a single reaction while still preserving the nested PCR approach described herein.

Referring to Fig. ID, a first (out-nested) round of PCR amplification can be performed on the top strand 18 and bottom strand 20 of the fragment 26. An out-nested region specific primer 130 is complementary to a sequence 122 downstream of ROI 22 and can be used to selectively enrich for the ROI 22. In one aspect, the region specific primer 130 can be used to target only one strand (e.g., top strand 18) of the fragment 26. The amplification product 134 (including top strand 134a and bottom strand 134b) following the first round extension with the region specific primer 130 can be denatured to provide a template strand (i.e., bottom strand 134b) for the label-specific primer 36 having the tail sequence 38. Notably, ROI 22 can only be exponentially amplified if the fragment 26 includes the ROI 22 proximal to the polynucleotide 14 to ultimately provide the amplification product 140 (including top strand 140a and bottom strand 140b).

Referring to Fig. IE, a second (in-nested) round of PCR amplification including the top strand 140a of the amplification product 140 can be performed with the region specific primer 30 including the tail sequence 32 which is complementary to the ROI 22. In one aspect, exponential amplification can be achieved through the inclusion of a suitable second primer, such as the label-specific primer 36. The in-nested round of PCR selectively enriches for the ROI 22. Following the second round extension with the primer 30, the amplification product 142 (including top strand 142a and bottom strand 142b) can be denatured to provide a template strand (i.e., bottom strand 142a) for the label specific first primer 42. Accordingly, the amplification product 142 is further amplified in a final round of PCR using the first primer 42 complementary to at least a portion of the tail sequence 32 added by the region specific primer 30, and second primer 44 complementary to at least a portion of the sequence 38 added by the label specific primer 36. The first primer 42 can also include a tail sequence 46. In one aspect, the tail sequence 38 and the tail sequence 46 can include labels, adapters, or indexes for platform specific sequencing. The resulting enriched amplification product 148 can then be sequenced. As discussed above, the second (in-nested) round and the final round of PCR illustrated in Fig. IE can be combined into a single round of PCR. Moreover, while the amplification product 48 and the amplification product 148 can be similar or identical depending on the design of the primers used in the workflow, the nested strategy used to prepare the amplification product 148 can provide for improved specificity and a higher on-target rate.

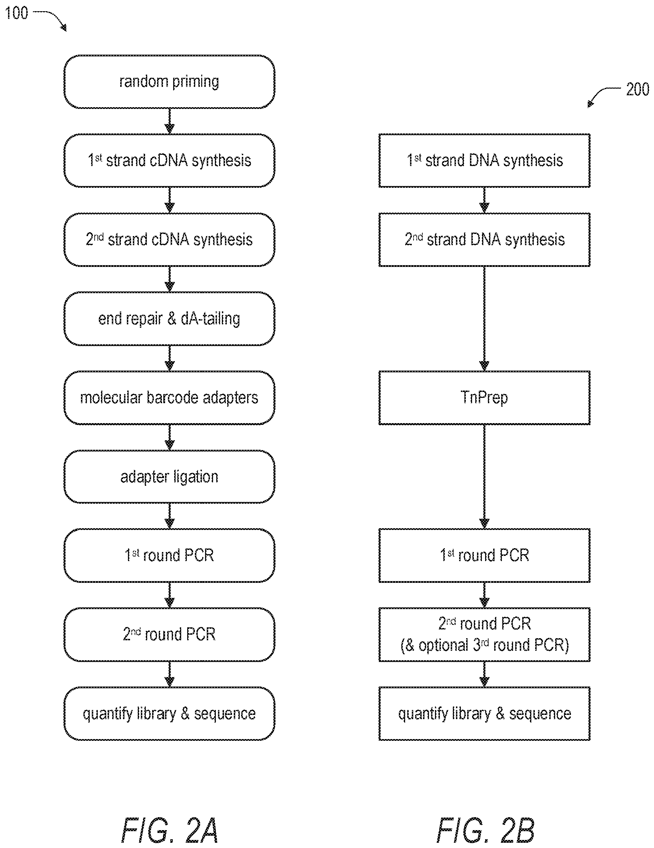

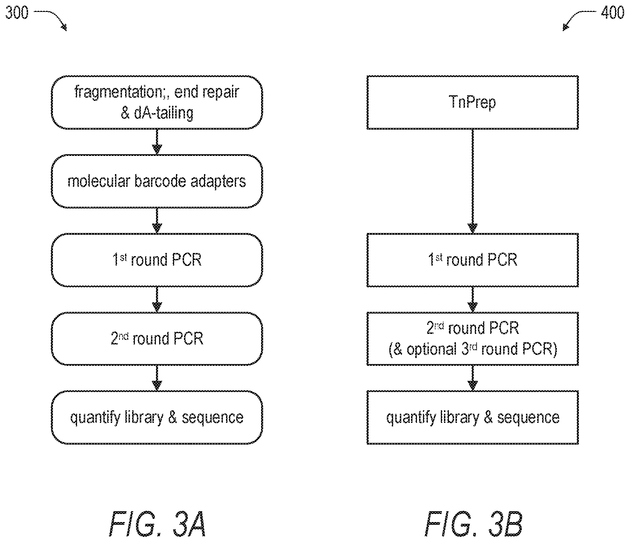

Turning to Fig. 2A, an example of a standard library preparation method 100 is shown. In comparison, Fig. 2B shows a method 200 according to the present disclosure that includes transposase-mediated amplicon sequencing or TnPrep. Notably, the method 200 includes fewer steps overall as compared with the method 100. Moreover, the method 200 excludes several steps included in the method 100, such as end repair, dA-tailing, and adapter ligation. In another example shown in Figs. 3A and 3B, a method 300 for library preparation is compared with a method 400 for transposase-mediated amplicon sequencing or TnPrep according to the present disclosure. Again, the method 400 includes fewer steps overall as compared with the method 300. Further, the method 400 excludes several steps included in the method 300, such as end repair and dA-tailing.

### EXAMPLES

### Example 1

Libraries were prepared from 50 ng Coriell DNA (NA12878) with a mix of 3 target specific primers. These libraries were sequenced to verify enrichment for targets of interest versus, for example, off-target amplification.

Target specific primers were used to amplify 1 ng of the final library to check that enrichment for the region of interest had taken place by qPCR and end-point PCR. Results from qPCR and Bioanalyzer traces are shown in Fig. 4 and Figs. 5-6, respectively.

As shown in Fig. 4, real-time amplification using primers specific to region of interest shows approximately 1000-fold enrichment for region of interest in the modified TnPrep library of the present disclosure as compared to the standard TnPrep library. The standard TnPrep library is a library prepared with the standard transposase loaded with R1 and R2 arms and amplified with the standard NEXTERA i5 and i7 primers, as opposed to the modified TnPrep library which is prepared with a transposase loaded with only R1 arms and amplified with NEXTERA i5 and target specific primers.

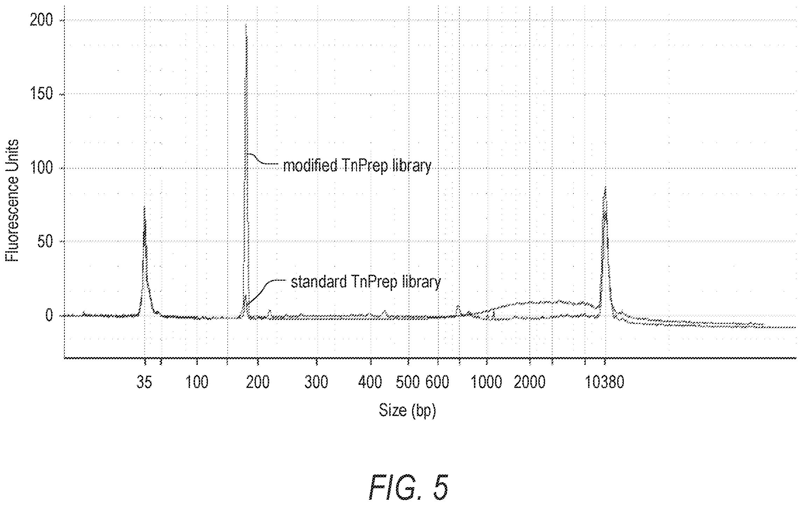

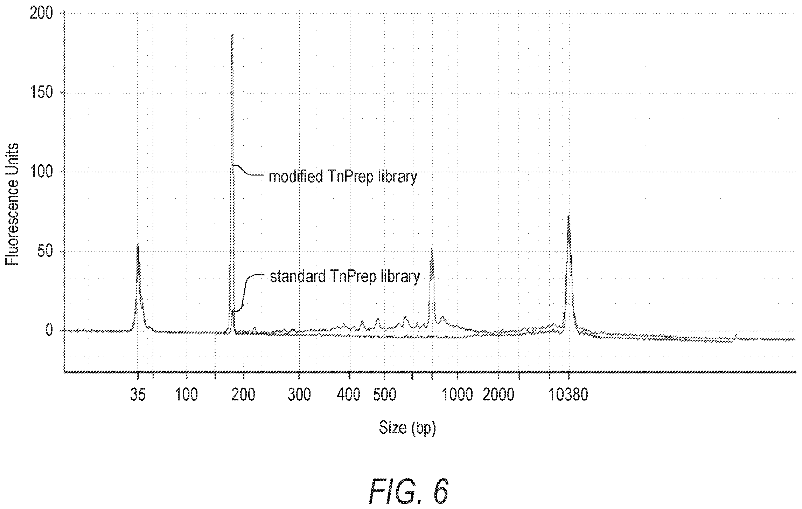

Figures 5 and 6 show traces obtained with an AGILENT Bioanalyzer after amplification of libraries with gene specific primers, showing enrichment of region of interest. Traces are from TnPrep libraries prepared with the combination of adapter primer and ROI primer, and from standard TnPrep libraries. Libraries were prepared with a final transposase concentration of either 36 µg/mL (Fig. 5) or 4 µg/mL (Fig. 6).

For the above example and a final transposase concentration of 36 µg/mL, libraries were prepared with pre-loaded transposase containing only R1 arms from 50 ng Coriell DNA (NA12878) as shown in Table 1.

**Table 1:**

| **Component** | **Volume (µL)** | **Final Concentration** |
|---|---|---|
| DNA | 4 | 2.5 ng/µL |
| Reaction buffer | 10 | 1x |
| MnCl₂ | 2 | 10 mM |
| Transposase (0.18 mg/ml) | 4 | 36 µg/mL |
| Total | 20 | n/a |

Samples were vortexed, spun down and incubated at 55° C for 5 min.

After incubation, 20 µL GHCl stop solution was added, and the samples were again vortexed, centrifuged and incubated at room temperature for 5 min.

Clean up reactions were carried out with 40 µL SPRI® beads and samples were resuspended to a total volume of 20 µL.

Samples were then heat treated at 95° C for 5 min to denature strands and prevent nick translation in the subsequent amplification step.

An amplification reaction was set up as shown in Table 2, and samples were cycled in a thermal cycler according the protocol shown in Table 3.

**Table 2:**

| **Component** | **Volume (µL)** |
|---|---|
| DNA | 10 |
| 5x ReadyMix | 4 |
| i5 primer (0.67 µM) | 3 |
| Reverse primer(s) (0.67 µM) | 3 |
| Total | 20 |

**Table 3:**

| **Step** | **Temperature (deg C)** | **Time (sec)** |
|---|---|---|
| 1 | 99 | 60 |
| 2 | 99 | 15 |
| 3 | 64 | 240 |
| Repeat steps 2-3 for a total of 15 cycles | | |

After amplification, samples were cleaned up with 40 µL SPRI beads and resuspended to a final volume of 20 µL. Samples were prepared for a second round of amplification as described in Table 4, and cycled in a thermal cycler according to the protocol shown in Table 5.

**Table 4:**

| **Component** | **Volume (µL)** |
|---|---|
| DNA | 10 |
| PI primer (2.5 µM) | 2 |
| Reverse primer (2.5 µM) | 2 |
| Hifi HotStart ReadyMix | 25 |
| Water | 11 |
| Total | 50 |

**Table 5:**

| **Step** | **Temperature (deg C)** | **Time (sec)** |
|---|---|---|
| 1 | 98 | 60 |
| 2 | 98 | 15 |
| 3 | 62 | 30 |
| 4 | 72 | 30 |
| Repeat steps 2-4 for a total of 8 - 10 cycles | | |
| 5 | 72 | 60 |

Following the second round of amplifications, samples were cleaned up with 50 µL SPRI® beads and resuspended in 20 µL of water. Samples were prepared for a final round of amplification as described in Table 6, and cycled in a thermocycler as described in Table 5.

**Table 6**

| **Component** | **Volume (µL)** |
|---|---|
| DNA | 10 |
| PI primer (2.5 µM) | 5 |
| 17 primer (2.5 µM) | 5 |
| Hifi HotStart ReadyMix | 25 |
| Water | 5 |
| Total | 50 |

Following the final round of amplifications, samples were cleaned up with 50 µL SPRI® beads and resuspended in 20 µL of water.

The oligo sequences used in the above examples are described as follows:
Transposase arms:
   Read1 End (R1): TCGTCGGCAGCGTCAGATGTGTATAAGAGACAG (SEQ ID NO: 1).
Amplification primers:
   NEXTERA amplification primer (S517): AATGATACGGCGACCACCGAGATCTACACGCGTAAGATCGTCGGCAGCGTC (SEQ ID NO: 2).

The three outer target specific reverse primers were as follows:
CTTCTCCACTTAATAAGCAGTTGAT (SEQ ID NO: 3);
TAAGGTATCAATAAATACTCACCAATCTTC (SEQ ID NO: 4); and
CCAGGCTGCCTCACCAT (SEQ ID NO: 5).

The three target specific reverse primers with out-nested portion of adapter sequence were as follows: GTCTCGTGGGCTCGGAGATGTGTATAAGAGACAGGTCCTGACTGTGGCGTCAT (SEQ ID NO: 8).

The sequence of the NEXTERA amplification primer (N70x) was as follows (the 17 representing an index tag):
CAAGCAGAAGACGGCATACGAGAT(I7)GTCTCGTGGGCTCGG (SEQ ID NO: 9).
PI primer: AAT GAT ACG GCG ACC ACC G*A (SEQ ID NO: 16)

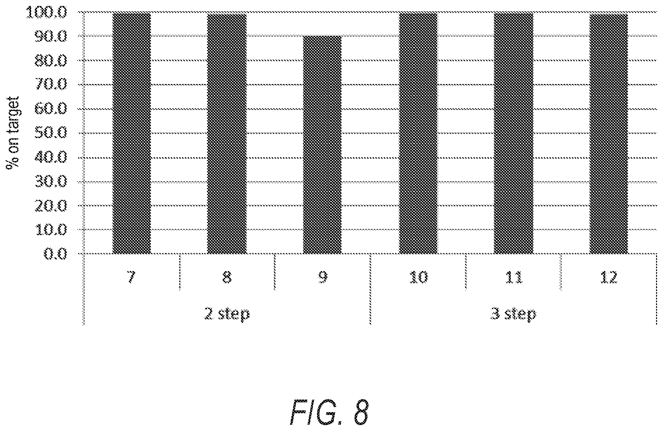

Successful enrichment of targeted regions of interest was confirmed by real time PCR (Fig. 7) and sequencing (Fig. 8).

### Example 2

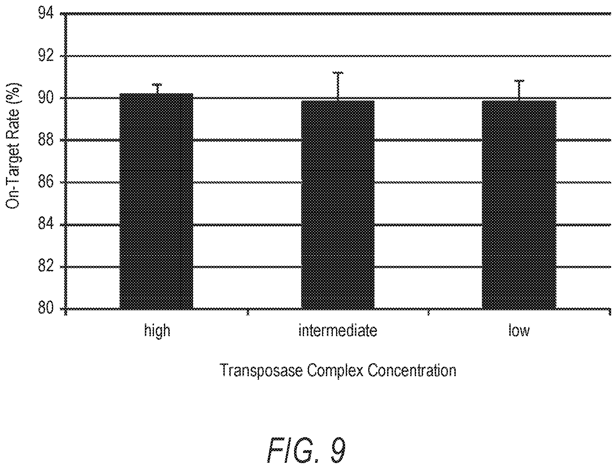

The effects of the concentration of transposase complex on the final insert size distribution, as well as metrics including on-target rate, were investigated by incubating 50 ng of human genomic DNA (Coriell, NA24385) with increasing quantities of transposase complex. Specifically, 50 ng of human genomic DNA (Coriell, NA24385) were incubated with one of a high concentration (36 µg/mL), an intermediate concentration (9 µg/mL), or a low concentration (1.125 µg/mL) of transposase complex. The highest concentration of transposase complex resulted in a greater abundance of shorter nucleic acid fragments (having an average fragment size of 550 nucleotides), whereas the lowest concentration of transposase complex resulted in larger nucleic acid fragments (having an average fragment size of 614 nucleotides). The intermediate concentration of transposase complex resulted in an average fragment size of 580 nucleotides. Moreover, nucleic acid libraries prepared with three different transposase complex concentrations exhibited similar on-target rates with no significant difference between the three concentrations (Fig. 9), which was indicative that the on-target rate is independent of insert size within the range tested.

### Example 3

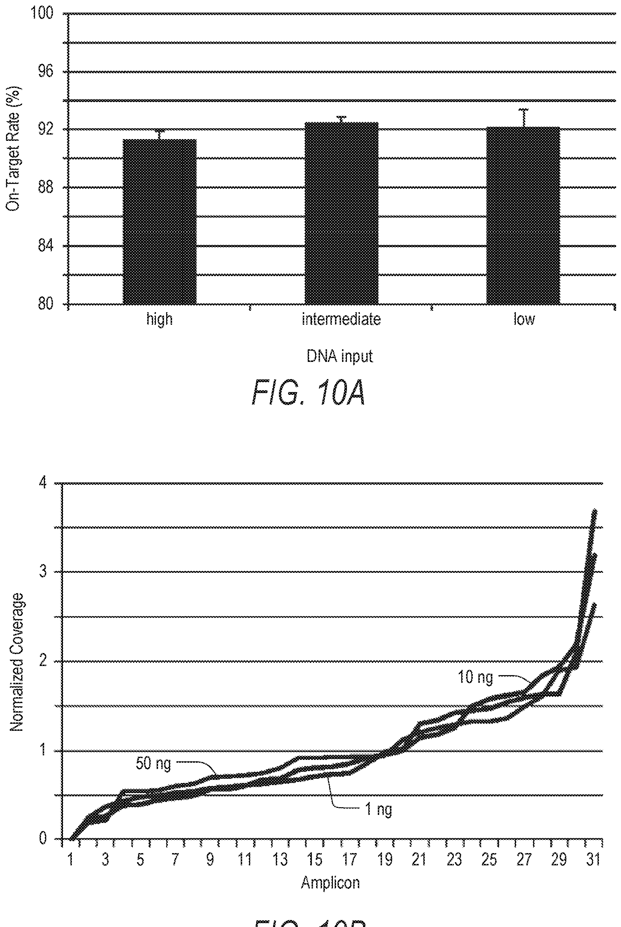

Turning to Figs. 10A and 10B, the impact of lowering the DNA input into the TnPrep workflow was investigated using the Quantitative Multiplex Reference Standard (HORIZON DISCOVERY HD701). Similar on-target rates were observed with decreasing DNA input from 50 ng to 1 ng, with all on-target rates calculated to be greater than 90% (in accordance with Fig. 10A, the high level of DNA input corresponds to 50 ng, the intermediate level of DNA input corresponds to 10 ng and the low level of DNA input corresponds to 1 ng). Notably, coverage uniformity was unaffected by decreasing DNA input. As shown in Table 7, the percentage of bases covered at a depth of at least 0.2x of the mean was greater than 94% for each of the tested DNA input amounts.

**Table 7**

| **DNA input (ng)** | **Bases covered at ≥ 0.2x of mean (%)** |
|---|---|
| 50 | 94.2 |
| 10 | 96.1 |
| 1 | 94.9 |

### Example 4

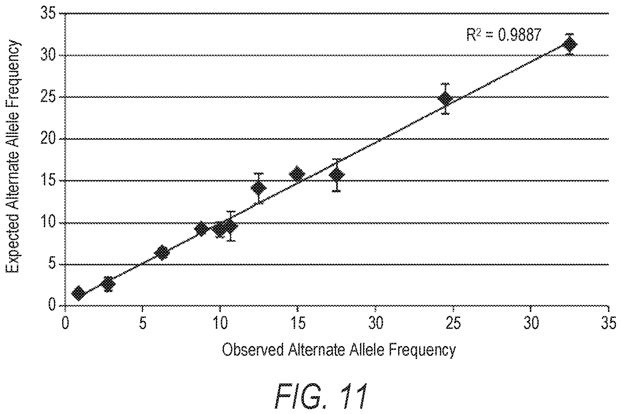

To investigate the ability of the disclosed methods to detect mutations, 11 single nucleotide variants (SNVs) and 2 deletions present in a Quantitative Multiplex Reference Standard (HORIZON DISCOVERY HD701) were targeted by a primer panel. The SNVs and deletions are known to be present at frequencies ranging from 0.9% up to 32.5%. Libraries were also constructed from a Structural Reference Standard (HORIZON DISCOVERY HD753), containing 2 gene fusions. With reference to Fig. 11, there was a strong correlation (R² = 0.9887) between observed and expected allele frequencies in the 50 ng libraries prepared from the Quantitative Multiplex Reference Standard (HORIZON DISCOVERY HD701). The libraries prepared from 10 ng of the Quantitative Multiplex Reference Standard (HORIZON DISCOVERY HD701) exhibited a similarly strong correlation between observed and expected allele frequencies, while the 1 ng libraries performed slightly worse (data not shown). Variants were detected using a previously published method (Wilm A. et al., 2012. Nucleic Acids Res. Dec; 40(22): 11189-201).

### Example 5

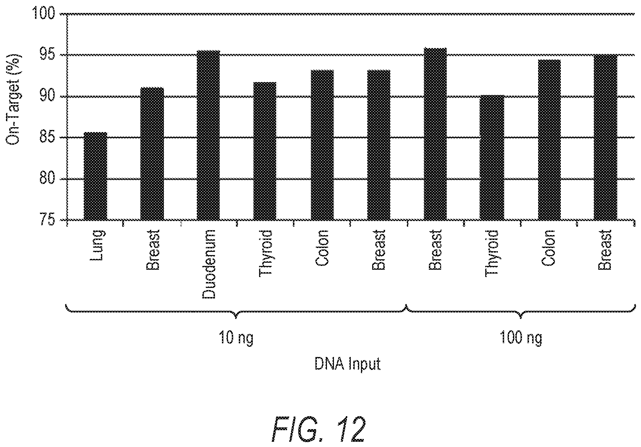

The performance of the disclosed method was further tested on a range of formalin-fixed paraffin embedded tissue (FFPET) samples from different tissue types and of varying quality. With reference to Fig. 12, on-target rates for nucleic acid libraries constructed from FFPE DNA were generally observed to be at least 90%, with the exception of a single library. No substantial difference in on-target rates was observed for libraries prepared from 10 ng of FFPE DNA as compared to libraries prepared from 100 ng of FFPE DNA. Notably, libraries derived from high quality DNA exhibited greater coverage uniformity than libraries constructed from FFPE (see Table 8).

**Table 8**

| **Tissue Sample** | **Bases covered at ≥ 0.2x of mean (%)** |
|---|---|
| Colon | 72.4 |
| Breast | 81.8 |
| Lung | 84.9 |
| Breast | 70.3 |
| Thyroid | 79.3 |

### Example 6

In this example, molecular barcodes (UIDs) were incorporated into the R1 arm in place of the i5 index as shown for the standard-length arm (standard R1 (SEQ ID NO: 10) and complementary sequence (SEQ ID NO: 11); 15 Primer (SEQ ID NO:12, with index sequence underlined)) as well as for the long R1 arm (long R1 arm (SEQ ID NO: 13, with UID sequence underlined and bolded) and complementary sequence (SEQ ID NO: 14); 15 Primer (SEQ ID NO:15)):
***Standard R1*** (5' to 3') =*TCGTCGGCAGCGTC***AGATGTGTATAAGAGACAG**
(3' to 5') = TCTACACATATTCTCTGTC
15 Primer (5' to 3') = AATGATACGGCGACCACCGAGATCTACACTAGATCGC*TCGTCGGCAGCGTC*

### Long Arm R1 with UID in place of i5 index:

***Long R1 Arm*** (5' to 3') = GACCACCGAGATCTACAC**NNNNNNNN***TCGTCGGCAGCGTC***AGATGTGTATAAGAGACAG** (3' to 5')= TCTACACATATTCTCTGTC 15 Primer (5' to 3') =AATGATACGGCGACCACCGAGATCTACAC

UID sequences of the disclosure may comprise or consist of 8 bases (e.g. 8 degenerate bases), which are preferably incorporated into one or both arms of the transposase. Preferably, the UID sequence replaces an index sequence (e.g. an i5 index sequence), as shown above. The incorporation of a UID sequence into an arm of the transposase facilitates the resolution of duplicates, enabling the generation of a consensus sequence for each original fragment of the resultant library. Generation of a consensus sequence for each original fragment of the resultant library using the UID sequences enables detection of true and/or rare variants in the library with an increased sensitivity compared to the use of a transposase lacking the UID sequences or a method lacking the consensus sequence for each original fragment of the resultant library.

For the above example and a final transposase concentration of 7.2 µg/mL, libraries were prepared with pre-loaded transposase containing only R1 arms from 10 ng Coriell DNA (NA12878) as shown in Table 9.

**Table 9.**

| **Component** | **Volume (µL)** | **Final Concentration** |
|---|---|---|
| DNA | 4 | 2.5 ng/µL |
| Reaction buffer | 10 | 1x |
| MnCl₂ | 2 | 2 mM |
| Transposase (0.036 mg/ml) | 4 | 7.2 µg/mL |
| Total | 20 | n/a |

Samples were vortexed, spun down and incubated at 55° C for 5 min.

After incubation, 20 µL GHCl stop solution was added, and the samples were again vortexed, centrifuged and incubated at room temperature for 5 min.

Clean up reactions were carried out with 40 µL SPRI® beads and samples were resuspended to a total volume of 11 µL.

Some samples were then heat treated at 95° C for 5 min to denature strands and prevent nick translation in the subsequent amplification step. Alternatively, some samples were not heat-treated and a nick-translation step was added to the subsequent amplification step. In some embodiments, the nick-translation step comprises an incubation of the amplification reaction at 72°C for 3 minutes. The inclusion of the nick-translation step produces an amplification product having an adapter on both the 5' and 3' ends of the fragments, as opposed to only the 5' end of each fragment in the absence of the nick-translation step.

An amplification reaction was set up as shown in Table 10, and samples were cycled in a thermal cycler according to the protocol shown in Table 3.

| **Component** | **Volume (µL)** |
|---|---|
| DNA | 10 |
| 5x ReadyMix | 4 |
| i5 primer (2.5 µM) | 2 |
| Reverse primers (1 µM) | 2 |
| Total | 20 |

After amplification, samples were cleaned up with 40 µL SPRI beads and resuspended to a final volume of 11 µL. Samples were prepared for a second round of amplification as described in Table 11, and cycled for 16 cycles in a thermal cycler according to the protocol shown in Table 5.

| **Component** | **Volume (µL)** |
|---|---|
| PCR product | 10 |
| Hifi HotStart ReadyMix | 25 |
| P1 primer (2.5 µM) | 5 |
| Reverse primers (2.5 µM) | 2 |
| 17 primer (2.5 µM) | 5 |
| Total | 50 |

Amplification primers (wherein the (*) in a primer sequence represents a phosphorothioate bond):
15 primer: AATGATACGGCGACCACCGAGATCTACAC (SEQ ID NO: 15)
P1 primer: AAT GAT ACG GCG ACC ACC G*A (SEQ ID NO: 16)

The 31 outer target specific reverse primers were as follows:

| | |
|---|---|
| CTTCTCCACTTAATAAGCAGTTGA*T | SEQ ID NO: 3 |
| TAAGGTATCAATAAATACTCACCAATCTT*C | SEQ ID NO: 4 |
| CCAGGCTGCCTCACCA*T | SEQ ID NO: 5 |
| CAGAAATCCTAAATGGTGGAGT*C | SEQ ID NO: 17 |
| GGCTTGGGCAAAGGAAAT*A | SEQ ID NO: 18 |
| CAAGGAAGCAGGACACCAA*T | SEQ ID NO: 19 |
| **TCAAACATCATCTTGTGAAAC*A** | SEQ ID NO: 20 |
| CAGTTGGCTTACTGGAAGTTG* A | SEQ ID NO: 21 |
| **AATCGGTTTAGGAATACAATTCT*G** | SEQ ID NO: 22 |
| CCAGAATTATAGGAACTTGCTAACA* | SEQ ID NO: 23 |
| **GGTGGAGGTAATTTTGAAGC*A** | SEQ ID NO: 24 |
| CAAGGGGAAAGTGTAAATCA*A | SEQ ID NO: 25 |
| CCCCATGGAACTTACCAAG*C | SEQ ID NO: 26 |
| AAAACCATCTTTCGTTTCCTT*C | SEQ ID NO: 27 |
| CATTTTGGCCAGGATGA*T | SEQ ID NO: 28 |
| TTCAAAGCCATTTTTCCAG*A | SEQ ID NO: 29 |
| GTGGGAAGGCGGTGTT*G | SEQ ID NO: 30 |
| **CACAGCGTCTCCGAGTC*C** | SEQ ID NO: 31 |
| GCACAGTTCAGAGGATATTTAAG*C | SEQ ID NO: 32 |
| GTGCAGCCCTCAGGGAG*T | SEQ ID NO: 33 |
| **CACCCCCAGGATTCTTACAGAAAA*C** | SEQ ID NO: 34 |
| **CTGCCAGACATGAGAAAAGGTG*G** | SEQ ID NO: 35 |
| **AATATACAGCTTGCAAGGACTCTG*G** | SEQ ID NO: 36 |
| **CCAATATTGTCTTTGTGTTCCCGG*A** | SEQ ID NO: 37 |
| **TCTGCTTTATTTATTCCAATAGGTATGG*T** | SEQ ID NO: 38 |
| **AGTTGAAACTAAAAATCCTTTGCAG*G** | SEQ ID NO: 39 |
| **TAAACAATACAGCTAGTGGGAAGG*C** | SEQ ID NO: 40 |
| **AGTGTATTAACCTTATGTGTGACATG*T** | SEQ ID NO: 41 |
| **TGAGTGAAGGACTGAGAAAATCCC*T** | SEQ ID NO: 42 |
| **AGAAATTAGATCTCTTACCTAAACTCTTCA*T** | SEQ ID NO: 43 |
| **GTGGAATCCAGAGTGAGCTTTCAT*T** | SEQ ID NO: 44 |

The 40 outer target specific reverse primers were as follows:

| | |
|---|---|
| GTTAATCAACTGATGCAAACTCTT*G | SEQ ID NO: 45 |
| AAATAATGCTCCTAGTACCTGTAG*A | SEQ ID NO: 46 |
| TCCTTTAATACAGAATATGGGTAAAGA*T | SEQ ID NO: 47 |
| TGTAAACCTTGCAGACAAACT*C | SEQ ID NO: 48 |
| CCATGAGGCAGAGCATAC*G | SEQ ID NO: 49 |
| ACATCCTGGTAGCTGAGG*G | SEQ ID NO: 50 |
| GTCTTTTGGTTTTTCTTGATAGTATTAAT*G | SEQ ID NO: 51 |
| TTCTTCCTAAGTGCAAAAGATAAC*T | SEQ ID NO: 52 |
| CAGTGTTTCTTTTAAATACCTGTTAAGTT*T | SEQ ID NO: 53 |
| CCACAGTTGCACAATATCCTT*T | SEQ ID NO: 54 |
| TGCAGCAATTCACTGTAAAG*C | SEQ ID NO: 55 |
| CTAATGTATATATGTTCTTAAATGGCTAC*G | SEQ ID NO: 56 |
| CCAGGACCAGAGGAAACC*T | SEQ ID NO: 57 |
| AAATAGTTTAAGATGAGTCATATTTGTG*G | SEQ ID NO: 58 |
| CTGTGGGGTGGAGAGCT*G | SEQ ID NO: 59 |
| GGTCACACTTGTTCCCCA*C | SEQ ID NO: 60 |
| CCGTTTGATCTGCTCCC*T | SEQ ID NO: 61 |
| CTGGATGGTCAGCGCAC*T | SEQ ID NO: 62 |
| GTTATGATTTTGCAGAAAACAGATC*T | SEQ ID NO: 63 |
| ACTATAATTACTCCTTAATGTCAGCTTA*T | SEQ ID NO: 64 |
| TGAAAATGGTCAGAGAAACCTTTAT*C | SEQ ID NO: 65 |
| TGCAGTTGTTTACCATGATAAC*G | SEQ ID NO: 66 |
| CGAAATAACACAAATTTTTAAGGTTACTG*A | SEQ ID NO: 67 |
| TGATTACACAGTATCCTCGACA*T | SEQ ID NO: 68 |
| CTGAAAAGAGTGAAGGATATAGGATA*C | SEQ ID NO: 69 |
| ACTGTTCTTCCTCAGACATTC*A | SEQ ID NO: 70 |
| CCACAGAGAAGTTGTTGAGG*G | SEQ ID NO: 71 |
| GCCTTGTACTGCAGAGACA*A | SEQ ID NO: 72 |
| TGGTGATGGGCTTGGTC*C | SEQ ID NO: 73 |
| TACAGCGGAGAAGGGAGC*G | SEQ ID NO: 74 |
| CAGGTAGGATCCAGCCC*A | SEQ ID NO: 75 |
| ATCCCCCAAAGCCAACA*A | SEQ ID NO: 76 |
| GTTTGGGGGTGTGTGGT*C | SEQ ID NO: 77 |
| GGTGCTTCCCATTCCAG*G | SEQ ID NO: 78 |
| ACAGTCAAGAAGAAAACGG*C | SEQ ID NO: 79 |
| AGAGGAGCTGGTGTTGTT*G | SEQ ID NO: 80 |
| ACCGCTTCTTGTCCTGC*T | SEQ ID NO: 81 |
| GAGCAGTAAGGAGATTCCC*C | SEQ ID NO: 82 |
| AAATCGGTAAGAGGTGGG*C | SEQ ID NO: 83 |
| CCCCCTACTGCTCACCT*G | SEQ ID NO: 84 |

The 31 target specific reverse primers with out-nested portion of adapter sequence were as follows:

The 40 target specific reverse primers with out-nested portion of the adapter sequence were as follows:

| | |
|---|---|
| GTCTCGTGGGCTCGGAGATGTGTATAAGAGACAGTGAAAGCTGTACCATACCTG*T | SEQ ID NO: 113 |
| GTCTCGTGGGCTCGGAGATGTGTATAAGAGACAGAGGTTAATATCCGCAAATGACTT*G | SEQ ID NO: 114 |
| GTCTCGTGGGCTCGGAGATGTGTATAAGAGACAGAGATGATCCGACAAGTGAGA*G | SEQ ID NO: 115 |
| GTCTCGTGGGCTCGGAGATGTGTATAAGAGACAGTACGTCTCCTCCGACCA*C | SEQ ID NO: 116 |
| GTCTCGTGGGCTCGGAGATGTGTATAAGAGACAGCGCAGCCTGTACCCAGT*G | SEQ ID NO: 117 |
| GTCTCGTGGGCTCGGAGATGTGTATAAGAGACAGGCGGAAGATGAAGATTTCGGA*T | SEQ ID NO: 118 |
| GTCTCGTGGGCTCGGAGATGTGTATAAGAGACAGGTTAGCTCATTTTTGTTAATGGTG*G | SEQ ID NO: 119 |
| GTCTCGTGGGCTCGGAGATGTGTATAAGAGACAGTTAAACTTTTCTTTTAGTTGTGCTG*A | SEQ ID NO: 120 |
| GTCTCGTGGGCTCGGAGATGTGTATAAGAGACAGGTATGCAACATTTCTAAAGTTACCTA*C | SEQ ID NO: 121 |
| GTCTCGTGGGCTCGGAGATGTGTATAAGAGACAGAAGACCATAACCCACCACA*G | SEQ ID NO: 122 |
| GTCTCGTGGGCTCGGAGATGTGTATAAGAGACAGCTGGAAAGGGACGAACTGG*T | SEQ ID NO: 123 |
| GTCTCGTGGGCTCGGAGATGTGTATAAGAGACAGCGACCCAGTTACCATAGCA*A | SEQ ID NO: 124 |
| GTCTCGTGGGCTCGGAGATGTGTATAAGAGACAGAGAAAATGGAAGTCTATGTGATCAA*G | SEQ ID NO: 125 |
| GTCTCGTGGGCTCGGAGATGTGTATAAGAGACAGCATTTTAAATTTTCTTTCTCTAGGTGAA*G | SEQ ID NO: 126 |
| GTCTCGTGGGCTCGGAGATGTGTATAAGAGACAGGGTCTTGGCCGAGGTCT*C | SEQ ID NO: 127 |
| GTCTCGTGGGCTCGGAGATGTGTATAAGAGACAGCAGCACCATGGGCACGT*C | SEQ ID NO: 128 |
| GTCTCGTGGGCTCGGAGATGTGTATAAGAGACAGGAGAGGTGGAAAGCGAGA*G | SEQ ID NO: 129 |
| GTCTCGTGGGCTCGGAGATGTGTATAAGAGACAGCACTCTTGCCCACACCG*C | SEQ ID NO: 130 |
| GTCTCGTGGGCTCGGAGATGTGTATAAGAGACAGCTGTATTTATTTCAGTGTTACTTACCT*G | SEQ ID NO: 131 |
| GTCTCGTGGGCTCGGAGATGTGTATAAGAGACAGTTATATTCAATTTAAACCCACCTATAATG*G | SEQ ID NO: 132 |
| GTCTCGTGGGCTCGGAGATGTGTATAAGAGACAGGTATCAAAGAATGGTCCTGCA*C | SEQ ID NO: 133 |
| GTCTCGTGGGCTCGGAGATGTGTATAAGAGACAGACACAACACAAAATAGCCGTAT*A | SEQ ID NO: 134 |
| GTCTCGTGGGCTCGGAGATGTGTATAAGAGACAGTCTATTCTTTCCTTTGTAGTGTCC*A | SEQ ID NO: 135 |
| GTCTCGTGGGCTCGGAGATGTGTATAAGAGACAGTGATTTCTGTTTTTACCTCCTAAAGA*A | SEQ ID NO: 136 |
| GTCTCGTGGGCTCGGAGATGTGTATAAGAGACAGATCTTTAAAGAGAAATTTGCTAAAGCTGT*G | SEQ ID NO: 137 |
| GTCTCGTGGGCTCGGAGATGTGTATAAGAGACAGACGTGTTTTGATCAAAGAAGAG*G | SEQ ID NO: 138 |
| GTCTCGTGGGCTCGGAGATGTGTATAAGAGACAGAGCCTCACGTTGGTCCA*C | SEQ ID NO: 139 |
| GTCTCGTGGGCTCGGAGATGTGTATAAGAGACAGGGATGGCTAGGCGAGGA*G | SEQ ID NO: 140 |
| GTCTCGTGGGCTCGGAGATGTGTATAAGAGACAGAGGGACTAGGCGTGGGA*T | SEQ ID NO: 141 |
| GTCTCGTGGGCTCGGAGATGTGTATAAGAGACAGCCAAGCCCTAGGGTGGT*G | SEQ ID NO: 142 |
| GTCTCGTGGGCTCGGAGATGTGTATAAGAGACAGACGCTCTTCTCACTCATATC*C | SEQ ID NO: 143 |
| GTCTCGTGGGCTCGGAGATGTGTATAAGAGACAGAAGAAATCTTAGACGTAAGCCCCT*C | SEQ ID NO: 144 |
| GTCTCGTGGGCTCGGAGATGTGTATAAGAGACAGTCCCATACCCTCTCAGC*G | SEQ ID NO: 145 |
| GTCTCGTGGGCTCGGAGATGTGTATAAGAGACAGGGATGAGCTACCTGGAGGA*T | SEQ ID NO: 146 |
| GTCTCGTGGGCTCGGAGATGTGTATAAGAGACAGATTTTGAGTGTTAGACTGGAAAC*T | SEQ ID NO: 147 |
| GTCTCGTGGGCTCGGAGATGTGTATAAGAGACAGGGGCAGTGCTAGGAAAGA*G | SEQ ID NO: 148 |
| GTCTCGTGGGCTCGGAGATGTGTATAAGAGACAGTGCTTACCTCGCTTAGTG*C | SEQ ID NO: 149 |
| GTCTCGTGGGCTCGGAGATGTGTATAAGAGACAGGCCGGGGATGTGATGAG*A | SEQ ID NO: 150 |
| GTCTCGTGGGCTCGGAGATGTGTATAAGAGACAGCAGGGGTCAGAGGCAAG*C | SEQ ID NO: 151 |
| GTCTCGTGGGCTCGGAGATGTGTATAAGAGACAGAGGGCCACTGACAACCA*C | SEQ ID NO: 152 |

The sequence of the NEXTERA amplification primer (N70x) was as follows (the I7 representing an index tag):
CAAGCAGAAGACGGCATACGAGAT(I7)GTCTCGTGGGCTCGG (SEQ ID NO: 9).

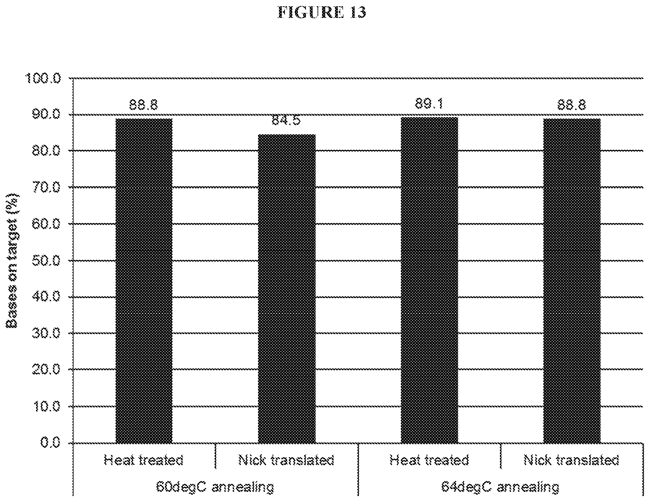

As shown in Fig. 13, the elimination of the heat denaturation step and inclusion of the nick-translation step maintained a comparable level of on-target amplification compared to the heat-denaturing step.

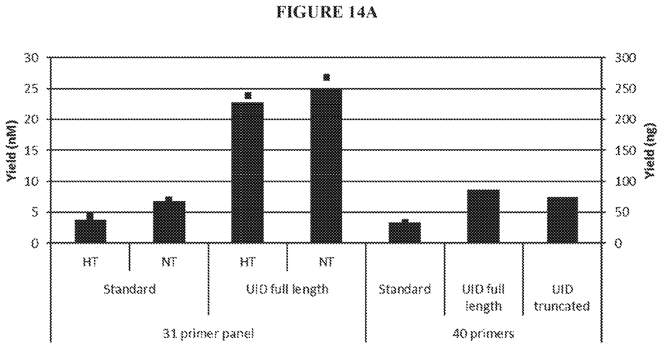

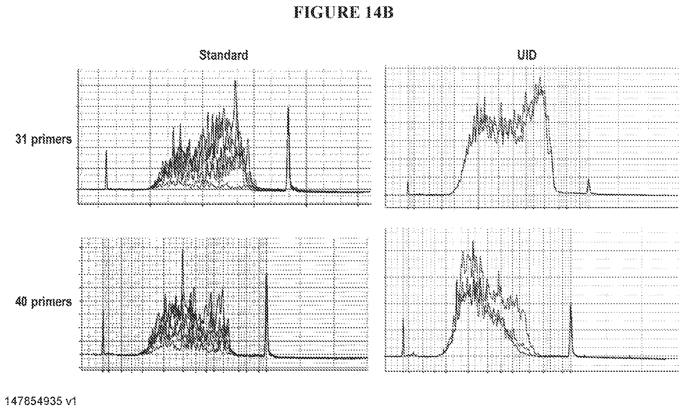

Figures 14A and 14B demonstrate that the including of the long R1 arm with the UID sequence may significantly improve yield compared to the use of a standard R1 arm.

### OTHER EMBODIMENTS

The schematic flow charts shown in the Figures are generally set forth as logical flow chart diagrams. As such, the depicted order and labeled steps are indicative of one embodiment of the presented method. Other steps and methods may be conceived that are equivalent in function, logic, or effect to one or more steps, or portions thereof, of the illustrated method. Additionally, the format and symbols employed in the Figures are provided to explain the logical steps of the method and are understood not to limit the scope of the method. Although various arrow types and line types may be employed, they are understood not to limit the scope of the corresponding method. Indeed, some arrows or other connectors may be used to indicate only the logical flow of the method. For instance, an arrow may indicate a waiting or monitoring period of unspecified duration between enumerated steps of the depicted method. Additionally, the order in which a particular method occurs may or may not strictly adhere to the order of the corresponding steps shown.

The present invention is presented in several varying embodiments in the following description with reference to the Figures, in which like numbers represent the same or similar elements. Reference throughout this specification to "one embodiment," "an embodiment," or similar language means that a particular feature, structure, or characteristic described in connection with the embodiment is included in at least one embodiment of the present invention. Thus, appearances of the phrases "in one embodiment," "in an embodiment," and similar language throughout this specification may, but do not necessarily, all refer to the same embodiment.

The described features, structures, or characteristics of the invention may be combined in any suitable manner in one or more embodiments. In the following description, numerous specific details are recited to provide a thorough understanding of embodiments of the system. One skilled in the relevant art will recognize, however, that the system and method may both be practiced without one or more of the specific details, or with other methods, components, materials, and so forth. In other instances, well-known structures, materials, or operations are not shown or described in detail to avoid obscuring aspects of the invention. Accordingly, the foregoing description is meant to be exemplary, and does not limit the scope of present inventive concepts.

## Claims

1. A method for targeted enrichment of nucleic acids, the method comprising:
contacting a nucleic acid including at least one region of interest with a plurality of transposase complexes, each of the transposase complexes including at least a transposase and a
first polynucleotide, the first polynucleotide having a transposon end sequence and a first label
sequence, the transposon end sequence corresponding to the first transposase;
incubating the nucleic acid and the transposase complexes under conditions whereby multiple transposon end sequences and associated label sequences are inserted into the nucleic
acid and the nucleic acid is fragmented into a plurality of nucleic acid fragments including first
polynucleotide attached to each 5' end of the nucleic acid fragments;
selectively amplifying the nucleic acid fragments, thereby enriching for a portion of the nucleic acid fragments including the at least one region of interest relative to a remaining portion
of the nucleic acid fragments; and
sequencing the enriched nucleic acid fragments.
wherein selectively amplifying the nucleic acid fragments further includes a first round of PCR with at least one region specific primer complementary to the
nucleic acid proximal to the at least one region of interest, and a label specific primer complementary to at least a portion of the first label sequence attached to each 5' end of the
nucleic acid fragments,
wherein selectively amplifying the nucleic acid fragments further
includes at least ten region specific primers, wherein each of the region specific primers are
complementary to the nucleic acid proximal to different regions of interest.

2. The method of claim 1, wherein selectively amplifying the nucleic acid fragments further
includes at least 100 region specific primers, wherein each of the region specific primers are
complementary to the nucleic acid proximal to different regions of interest.

3. The method of claim 1, wherein selectively amplifying the nucleic acid fragments further
includes at least 1,000 region specific primers, wherein each of the region specific primers are
complementary to the nucleic acid proximal to different regions of interest.

4. The method of claim 1, wherein selectively amplifying the nucleic acid fragments further
includes at least 10,000 region specific primers, wherein each of the region specific primers are
complementary to the nucleic acid proximal to different regions of interest.

5. The method of claim 1, wherein selectively amplifying the nucleic acid fragments further
includes at least 100,000 region specific primers, wherein each of the region specific primers are
complementary to the nucleic acid proximal to different regions of interest.

6. The method of claim 1, wherein the nucleic acid is a double-stranded DNA.

7. The method of claim 1, wherein the transposase complex includes two of the first polynucleotides.

8. The method of claim 1, wherein the first label sequence includes at least one of i) an adapter sequence, ii) a unique identifier sequence, and iii) and sample identifier sequence.

9. The method of claim 1, wherein the first label sequence includes an adapter sequence.

10. A kit for targeted enrichment of at least one region of interest within a nucleic acid, the
kit comprising:
a transposase;
a first polynucleotide having a transposon end sequence and a first label sequence, the transposon end sequence corresponding to the first transposase;
at least 10 region specific primers complementary to a nucleic acid proximal to at least 10 regions of interest, and a label specific primer complementary to at least a portion of the first
label sequence.

11. A kit for targeted enrichment of at least one region of interest within a nucleic acid, the
kit comprising:
a transposase complex comprising a transposase and a first polynucleotide having a transposon end sequence and a first label sequence, the transposon end sequence corresponding
to the first transposase;
at least 10 region specific primers complementary to a nucleic acid proximal to at least 10 regions of interest, and a label specific primer complementary to at least a portion of the first
label sequence.

12. A method for targeted enrichment of nucleic acids, the method comprising:
contacting a nucleic acid including at least one region of interest with a plurality of transposase complexes, each of the transposase complexes including at least a transposase and a
first polynucleotide, the first polynucleotide having a transposon end sequence and a first label
sequence, the transposon end sequence corresponding to the first transposase;
incubating the nucleic acid and the transposase complexes under conditions whereby multiple transposon end sequences and associated label sequences are inserted into the nucleic
acid and the nucleic acid is fragmented into a plurality of nucleic acid fragments including first
polynucleotide attached to each 5' end of the nucleic acid fragments;
performing a first round of amplification including the nucleic acid fragments and at least
a first sequence specific primer that is out-nested relative to the region of interest, thereby
yielding a first amplification product;
performing a second round of amplification including the first amplification product and
at least a second sequence specific primer that is in-nested relative to first sequence specific
primer, thereby yielding a second amplification product, thereby enriching for a portion of the
nucleic acid fragments including the at least one region of interest relative to a remaining portion
of the nucleic acid fragments; and
sequencing the enriched nucleic acid fragments including the second amplification product.

## Patentansprüche

1. Verfahren zur gezielten Anreicherung von Nukleinsäuren, wobei das Verfahren Folgendes umfasst:
Inkontaktbringen einer Nukleinsäure, die mindestens eine Region von Interesse einschließt, mit einer Vielzahl von Transposasekomplexen, wobei jeder der Transposasekomplexe mindestens eine Transposase und ein erstes Polynukleotid einschließt, wobei das erste Polynukleotid eine Transposonendsequenz und eine erste Markierungssequenz aufweist, wobei die Transposonendsequenz der ersten Transposase entspricht;
Inkubieren der Nukleinsäure und der Transposasekomplexe unter Bedingungen, unter denen mehrere Transposonendsequenzen und assoziierte Markierungssequenzen in die Nukleinsäure eingebracht werden und die Nukleinsäure in eine Vielzahl von Nukleinsäurefragmenten fragmentiert wird, die das an jedes 5'-Ende der Nukleinsäurefragmente angelagerte erste Polynukleotid einschließen;
selektives Amplifizieren der Nukleinsäurefragmente, wodurch ein Teil der Nukleinsäurefragmente, die die mindestens eine Region von Interesse einschließen, in Bezug auf einen verbleibenden Teil der Nukleinsäurefragmente angereichert wird; und
Sequenzieren der angereicherten Nukleinsäurefragmente,
wobei das selektive Amplifizieren der Nukleinsäurefragmente ferner eine erste PCR-Runde mit mindestens einem regionspezifischen Primer, der zu der Nukleinsäure proximal zu der mindestens einen Region von Interesse komplementär ist, und einem markierungsspezifischen Primer, der zu mindestens einem Abschnitt der an jedes 5'-Ende der Nukleinsäurefragmente angelagerten ersten Markierungssequenz komplementär ist, einschließt,
wobei das selektive Amplifizieren der Nukleinsäurefragmente ferner mindestens zehn regionspezifische Primer einschließt, wobei jeder der regionspezifischen Primer zu der Nukleinsäure proximal zu verschiedenen Regionen von Interesse komplementär ist.

2. Verfahren nach Anspruch 1, wobei das selektive Amplifizieren der Nukleinsäurefragmente ferner mindestens 100 regionspezifische Primer einschließt, wobei jeder der regionspezifischen Primer zu der Nukleinsäure proximal zu verschiedenen Regionen von Interesse komplementär ist.

3. Verfahren nach Anspruch 1, wobei das selektive Amplifizieren der Nukleinsäurefragmente ferner mindestens 1.000 regionspezifische Primer einschließt, wobei jeder der regionspezifischen Primer zu der Nukleinsäure proximal zu verschiedenen Regionen von Interesse komplementär ist.

4. Verfahren nach Anspruch 1, wobei das selektive Amplifizieren der Nukleinsäurefragmente ferner mindestens 10.000 regionspezifische Primer einschließt, wobei jeder der regionspezifischen Primer zu der Nukleinsäure proximal zu verschiedenen Regionen von Interesse komplementär ist.

5. Verfahren nach Anspruch 1, wobei das selektive Amplifizieren der Nukleinsäurefragmente ferner mindestens 100.000 regionspezifische Primer einschließt, wobei jeder der regionspezifischen Primer zu der Nukleinsäure proximal zu verschiedenen Regionen von Interesse komplementär ist.

6. Verfahren nach Anspruch 1, wobei die Nukleinsäure eine doppelsträngige DNA ist.

7. Verfahren nach Anspruch 1, wobei der Transposasekomplex zwei von den ersten Polynukleotiden einschließt.

8. Verfahren nach Anspruch 1, wobei die erste Markierungssequenz mindestens eines von i) einer Adaptorsequenz, ii) einer nur einmal vorhandenen Identifikationssequenz und iii) einer Probenidentifikationssequenz einschließt.

9. Verfahren nach Anspruch 1, wobei die erste Markierungssequenz eine Adaptorsequenz einschließt.

10. Kit zur gezielten Anreicherung von mindestens einer Region von Interesse in einer Nukleinsäure, wobei der Kit Folgendes umfasst:
eine Transposase;
ein erstes Polynukleotid mit einer Transposonendsequenz und einer ersten Markierungssequenz, wobei die Transposonendsequenz der ersten Transposase entspricht;
mindestens 10 regionspezifische Primer, die zu einer Nukleinsäure proximal zu mindestens 10 Regionen von Interesse komplementär sind, und
einen markierungsspezifischen Primer, der zu mindestens einem Abschnitt der ersten Markierungssequenz komplementär ist.

11. Kit zur gezielten Anreicherung von mindestens einer Region von Interesse in einer Nukleinsäure, wobei der Kit Folgendes umfasst:
einen Transposasekomplex, umfassend eine Transposase und ein erstes Polynukleotid mit einer Transposonendsequenz und einer ersten Markierungssequenz, wobei die Transposonendsequenz der ersten Transposase entspricht;
mindestens 10 regionspezifische Primer, die zu einer Nukleinsäure proximal zu mindestens 10 Regionen von Interesse komplementär sind, und
einen markierungsspezifischen Primer, der zu mindestens einem Abschnitt der ersten Markierungssequenz komplementär ist.

12. Verfahren zur gezielten Anreicherung von Nukleinsäuren, wobei das Verfahren Folgendes umfasst:
Inkontaktbringen einer Nukleinsäure, die mindestens eine Region von Interesse einschließt, mit einer Vielzahl von Transposasekomplexen, wobei jeder der Transposasekomplexe mindestens eine Transposase und ein erstes Polynukleotid einschließt, wobei das erste Polynukleotid eine Transposonendsequenz und eine erste Markierungssequenz aufweist, wobei die Transposonendsequenz der ersten Transposase entspricht;
Inkubieren der Nukleinsäure und der Transposasekomplexe unter Bedingungen, unter denen mehrere Transposonendsequenzen und assoziierte Markierungssequenzen in die Nukleinsäure eingebracht werden und die Nukleinsäure in eine Vielzahl von Nukleinsäurefragmenten fragmentiert wird, die das an jedes 5'-Ende der Nukleinsäurefragmente angelagerte erste Polynukleotid einschließen;
Durchführen einer ersten Amplifikationsrunde, die die Nukleinsäurefragmente und mindestens einen ersten sequenzspezifischen Primer einschließt, der bezogen auf die Region von Interesse außerhalb verschachtelt ist, wodurch ein erstes Amplifikationsprodukt erhalten wird;
Durchführen einer zweiten Amplifikationsrunde, die das erste Amplifikationsprodukt und mindestens einen zweiten sequenzspezifischen Primer einschließt, der in Bezug auf den ersten sequenzspezifischen Primer innerhalb verschachtelt ist, wodurch ein zweites Amplifikationsprodukt erhalten wird, wodurch ein Teil der Nukleinsäurefragmente, die die mindestens eine Region von Interesse einschließen, bezogen auf einen verbleibenden Teil der Nukleinsäurefragmente angereichert wird; und
Sequenzieren der angereicherten Nukleinsäurefragmente, die das zweite Amplifikationsprodukt einschließen.

## Revendications

1. Procédé d'enrichissement ciblé d'acides nucléiques, le procédé comprenant :
la mise en contact d'un acide nucléique comprenant au moins une région d'intérêt avec une pluralité de complexes de transposase, chacun des complexes de transposase comprenant au moins une transposase et un premier polynucléotide, le premier polynucléotide ayant une séquence d'extrémité de transposon et une première séquence de marqueur, la séquence d'extrémité de transposon correspondant à la première transposase ;
l'incubation de l'acide nucléique et des complexes de transposase dans des conditions par lesquelles de multiples séquences d'extrémité de transposon et séquences de marqueur associées sont insérées dans l'acide nucléique et l'acide nucléique est fragmenté en une pluralité de fragments d'acide nucléique comprenant un premier polynucléotide lié à chaque extrémité 5' des fragments d'acide nucléique ;
l'amplification sélective des fragments d'acide nucléique, pour ainsi enrichir une partie des fragments d'acide nucléique comprenant l'au moins une région d'intérêt par rapport à une partie restante des fragments d'acide nucléique ; et
le séquençage des fragments d'acide nucléique enrichis,
dans lequel l'amplification sélective des fragments d'acide nucléique comprend en outre un premier cycle de PCR avec au moins une amorce spécifique d'une région complémentaire de l'acide nucléique proximal de l'au moins une région d'intérêt, et une amorce spécifique d'un marqueur complémentaire d'au moins une partie de la première séquence de marqueur liée à chaque extrémité 5' des fragments d'acide nucléique,
dans lequel l'amplification sélective des fragments d'acide nucléique comprend en outre au moins dix amorces spécifiques d'une région, dans lequel chacune des amorces spécifiques d'une région est complémentaire de l'acide nucléique proximal de différentes régions d'intérêt.

2. Procédé selon la revendication 1, dans lequel l'amplification sélective des fragments d'acide nucléique comprend en outre au moins 100 amorces spécifiques d'une région, dans lequel chacune des amorces spécifiques d'une région est complémentaire de l'acide nucléique proximal de différentes régions d'intérêt.

3. Procédé selon la revendication 1, dans lequel l'amplification sélective des fragments d'acide nucléique comprend en outre au moins 1000 amorces spécifiques d'une région, dans lequel chacune des amorces spécifiques d'une région est complémentaire de l'acide nucléique proximal de différentes régions d'intérêt.

4. Procédé selon la revendication 1, dans lequel l'amplification sélective des fragments d'acide nucléique comprend en outre au moins 10 000 amorces spécifiques d'une région, dans lequel chacune des amorces spécifiques d'une région est complémentaire de l'acide nucléique proximal de différentes régions d'intérêt.

5. Procédé selon la revendication 1, dans lequel l'amplification sélective des fragments d'acide nucléique comprend en outre au moins 100 000 amorces spécifiques d'une région, dans lequel chacune des amorces spécifiques d'une région est complémentaire de l'acide nucléique proximal de différentes régions d'intérêt.

6. Procédé selon la revendication 1, dans lequel l'acide nucléique est un ADN bicaténaire.

7. Procédé selon la revendication 1, dans lequel le complexe de transposase comprend deux des premiers polynucléotides.

8. Procédé selon la revendication 1, dans lequel la première séquence de marqueur comprend au moins une parmi i) une séquence d'adaptateur, ii) une séquence d'identificateur unique et iii) une séquence d'identificateur d'échantillon.

9. Procédé selon la revendication 1, dans lequel la première séquence de marqueur comprend une séquence d'adaptateur.

10. Kit pour l'enrichissement ciblé d'au moins une région d'intérêt à l'intérieur d'un acide nucléique, le kit comprenant :
une transposase ;
un premier polynucléotide ayant une séquence d'extrémité de transposon et une première séquence de marqueur, la séquence d'extrémité de transposon correspondant à la première transposase ;
au moins 10 amorces spécifiques d'une région complémentaires d'un acide nucléique proximal d'au moins 10 régions d'intérêt et une amorce spécifique d'un marqueur complémentaire d'au moins une partie de la première séquence de marqueur.

11. Kit pour l'enrichissement ciblé d'au moins une région d'intérêt à l'intérieur d'un acide nucléique, le kit comprenant :
un complexe de transposase comprenant une transposase et un premier polynucléotide ayant une séquence d'extrémité de transposon et une première séquence de marqueur, la séquence d'extrémité de transposon correspondant à la première transposase ;
au moins 10 amorces spécifiques d'une région complémentaires d'un acide nucléique proximal d'au moins 10 régions d'intérêt et une amorce spécifique d'un marqueur complémentaire d'au moins une partie de la première séquence de marqueur.

12. Procédé d'enrichissement ciblé d'acides nucléiques, le procédé comprenant :
la mise en contact d'un acide nucléique comprenant au moins une région d'intérêt avec une pluralité de complexes de transposase, chacun des complexes de transposase comprenant au moins une transposase et un premier polynucléotide, le premier polynucléotide ayant une séquence d'extrémité de transposon et une première séquence de marqueur, la séquence d'extrémité de transposon correspondant à la première transposase ;
l'incubation de l'acide nucléique et des complexes de transposase dans des conditions par lesquelles de multiples séquences d'extrémité de transposon et séquences de marqueur associées sont insérées dans l'acide nucléique et l'acide nucléique est fragmenté en une pluralité de fragments d'acide nucléique comprenant un premier polynucléotide lié à chaque extrémité 5' des fragments d'acide nucléique ;
l'exécution d'un premier cycle d'amplification comprenant les fragments d'acide nucléique et au moins une première amorce spécifique d'une séquence qui est nichée à l'extérieur par rapport à la région d'intérêt, donnant ainsi un premier produit d'amplification ;
l'exécution d'un second cycle d'amplification comprenant le premier produit d'amplification et au moins une seconde amorce spécifique d'une séquence qui est nichée à l'intérieur par rapport à la première amorce spécifique d'une séquence, donnant ainsi un second produit d'amplification, pour ainsi enrichir une partie des fragments d'acide nucléique comprenant l'au moins une région d'intérêt par rapport à une partie restante des fragments d'acide nucléique ; et
le séquençage des fragments d'acide nucléique enrichis comprenant le second produit d'amplification.
